# EUROPÄISCHE PATENTANMELDUNG

(11) **EP 2 371 823 A1**
(43) Veröffentlichungstag der Anmeldung: **05.10.2011**
(21) Anmeldenummer: 10158949.7
(22) Anmeldetag: 01.04.2010
(51) Int. Cl.: C07D 249/12, A01N 43/653, C07C 311/16

(54) **Cyclopropyl-substituierte Phenylsulfonylamino(thio)carbonyltriazolinone, ihre Herstellung und Verwendung als Herbizide und Pflanzenwachstumsregulatoren**

(71) Anmelder: Bayer CropScience AG, 40789 Monheim (DE)
(72) Erfinder: Die Erfindernennung liegt noch nicht vor
(74) Vertreter: Immler, Dorian

(57) **Zusammenfassung**

Beschrieben werden Cyclopropyl-substituierte Phenylsulfonylamino(thio)-carbonyltriazolinone der allgemeinen Formel (I) und deren Salze 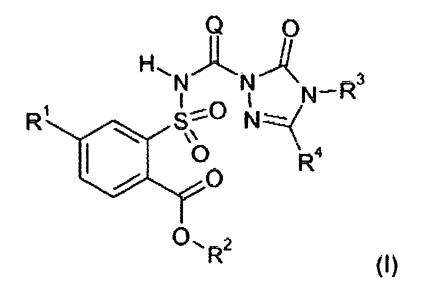 in welcher die jeweiligen Substituenten die in der Beschreibung angegebenen Bedeutungen aufweisen.
Die Cyclopropyl-substituierten Phenylsulfonylamino(thio)-carbonyltriazolinone der allgemeinen Formel (I) und/oder deren Salze können als Herbizide und Pflanzenwachstumsregulatoren verwendet werden.

## Beschreibung

Die Erfindung betrifft neue 4-Cyclopropyl-substituierte Phenylsulfonylamino(thio)-carbonyltriazolinone. Weiterer Gegenstand der vorliegenden Erfindung sind Mischungen der zuvor genannten Triazolinon-Derivate mit anderen Herbiziden und/oder Safenern. Darüber hinaus betrifft die vorliegende Erfindung Verfahren zur Herstellung der zuvor genannten Triazolinon-Derivate sowie die Verwendung als Herbizide und Pflanzenwachstumsregulatoren allein und in Mischung mit Safenern und/oder in Mischung mit anderen Herbiziden, insbesondere deren Verwendung zur Pflanzenbekämpfung in speziellen Pflanzenkulturen oder als Pflanzenschutzregulatoren.

Es sind herbizid wirksame 2-Alkoxycarbonyl-substituierte Phenylsulfonylamino(thio)-carbonyltriazolinone - auch mit weiteren Substituenten im Phenylring - bekannt (vgl. DE 4131842, DE 4343595, WO 1996/11188, WO 1996/27590, WO 1996/27591, WO 1996/35680, WO 1999/61429, WO 2003/02096, WO 2006/114221).

Die gemäß den oben erwähnten Schriften bereits bekannten Wirkstoffe weisen in ihrer Anwendung Nachteile auf, sei es,
(a) dass sie keine oder aber eine nur unzureichende herbizide Wirkung gegen Schadpflanzen besitzen,
(b) dass nur ein zu geringes Spektrum an Schadpflanzen bekämpft werden kann, oder
(c) dass sie eine zu geringe Selektivität in Nutzpflanzenkulturen besitzen.

Es ist deshalb wünschenswert, alternative chemische Wirkstoffe auf Basis von entsprechenden Triazolinon-Derivaten bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Daraus ergibt sich im Allgemeinen als Aufgabe der vorliegenden Erfindung, entsprechende alternative Triazolinon-Derivate bereitzustellen, welche als Herbizide oder Pflanzenwachstumsregulatoren, insbesondere mit einer zufrieden stellenden herbiziden Wirkung gegen Schadpflanzen, mit einem breiten Spektrum gegenüber Schadpflanzen und/oder mit einer hohen Selektivität in Nutzpflanzenkulturen, eingesetzt werden können. Diese Triazolinon-Derivate sollten dabei vorzugsweise ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen, als die aus dem Stand der Technik bekannten Triazolinon-Derivate haben.

Es wurden nun neue 4-Cyclopropyl-substituierte Phenylsulfonylamino(thio)carbonyltriazolinone der allgemeinen Formel (I), 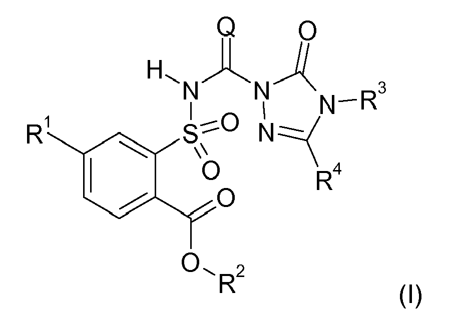 gefunden, in welcher
- R¹: für (R^{c})ₙ-Cyclopropyl steht, wobei der Cyclopropyl-Rest über die Position 1' mit dem Phenyl-Rest verknüpft ist, (R^{c})ₙ an den Positionen 1', 2' und 3' steht und wobei es sich, für alle n ≥ 2, bei R^{c} um gleiche oder verschiedene Reste handeln kann;
- R^{c}: ausgewählt ist aus der Gruppe, bestehend aus Halogen, Cyano und jeweils gegebenenfalls substituiertem Alkyl, Cycloalkyl, Alkenyl, Alkinyl, Alkoxy, Alkylcarbonyl oder Alkoxycarbonyl;
- R²: ausgewählt ist aus der Gruppe, bestehend aus jeweils gegebenenfalls substituiertem Alkyl, Alkenyl, Alkinyl, Cycloalkyl, Cycloalkylalkyl, Aryl, Arylalkyl, Heterocyclyl oder Heterocyclylalkyl;
- R³: ausgewählt ist aus der Gruppe, bestehend aus Wasserstoff, Hydroxy, Amino, Cyano, (C₂-C₁₀)-Alkylidenamino, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertem Alkyl mit 1 bis 6 Kohlenstoffatomen, jeweils gegebenenfalls durch Fluor, Chlor und/oder Brom substituiertem Alkenyl oder Alkinyl mit jeweils 2 bis 6 Kohlenstoffatomen, jeweils gegebenenfalls durch Fluor, Chlor, Brom, Cyano, (C₁-C₄)-Alkoxy oder (C₁-C₄)-Alkoxycarbonyl substituiertem Alkoxy, Alkylamino oder Alkyl-carbonylamino mit jeweils 1 bis 6 Kohlenstoffatomen in der Alkylgruppe, Alkenyloxy mit 3 bis 6 Kohlenstoffatomen, Dialkylamino mit jeweils 1 bis 4 Kohlenstoffatomen in den Alkylgruppen, jeweils gegebenenfalls durch Fluor, Chlor, Brom, Cyano und/oder (C₁-C₄)-Alkyl substituiertem Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder jeweils gegebenenfalls durch Fluor, Chlor, Brom, Cyano, Nitro, (C₁-C₄)-Alkyl, Trifluormethyl und/oder (C₁-C₄)-Alkoxy substituiertem Aryl oder Arylalkyl mit jeweils 6 oder 10 Kohlenstoffatomen in der Arylgruppe und gegebenenfalls 1 bis 4 Kohlenstoffatomen im Alkylteil;
- R⁴: ausgewählt ist aus der Gruppe, bestehend aus Wasserstoff, Hydroxy, Mercapto, Amino, Cyano, Fluor, Chlor, Brom, Iod, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Alkyl mit 1 bis 6 Kohlenstoffatomen, jeweils gegebenenfalls durch Fluor, Chlor und/oder Brom substituiertes Alkenyl oder Alkinyl mit jeweils 2 bis 6 Kohlenstoffatomen, jeweils gegebenenfalls durch Fluor, Chlor, Cyano, (C₁-C₄)-Alkoxy oder (C₁-C₄)-Alkoxycarbonyl substituiertes Alkoxy, Alkylthio, Alkylamino oder Alkyl-carbonylamino mit jeweils 1 bis 6 Kohlenstoffatomen in der Alkylgruppe, Alkenyloxy, Alkinyloxy, Alkenylthio, Alkinylthio, Alkenylamino oder Alkinylamino mit jeweils 3 bis 6 Kohlenstoffatomen in der Alkenyl- oder Alkinylgruppe, Dialkylamino mit jeweils 1 bis 4 Kohlenstoffatomen in den Alkylgruppen, jeweils gegebenenfalls durch Fluor, Chlor, Brom, Cyano und/oder (C₁-C₄)-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl-bzw. Cycloalkenylgruppe und gegebenenfalls 1 bis 4 Kohlenstoffatomen im Alkylteil oder jeweils gegebenenfalls durch Fluor, Chlor, Brom, Cyano, Nitro, (C₁-C₄)-Alkyl, Trifluormethyl, (C₁-C₄)-Alkoxy und/oder (C₁-C₄)-Alkoxycarbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil;
- Q: ausgewählt ist aus der Gruppe, bestehend aus Sauerstoff und Schwefel, bevorzugt Sauerstoff, und
- n: 0, 1, 2, 3, 4 oder 5.
bedeutet.

Von einer ersten Ausführungsform der vorliegenden Erfindung werden Verbindungen der allgemeinen Formel (I) umfasst, in welchen
- R^{c}: bevorzugt ausgewählt ist aus der Gruppe, bestehend aus Chlor, Brom, Fluor, Cyano, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkoxycarbonyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkylcarbonyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₂-C₆)-Alkenyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₂-C₆)-Alkinyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₃-C₆)-Cycloalkyl und gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkoxy;
- R^{c}: besonders bevorzugt ausgewählt ist aus der Gruppe, bestehend aus Chlor, Fluor, Cyano, gegebenenfalls durch Fluor, Chlor, Cyano, (C₁-C₄)-Alkyl, (C₁-C₄)-Alkoxy, (C₁-C₄)-Alkoxycarbonyl oder (C₃-C₆)-Cycloalkyl substituiertes (C₁-C₄)-Alkyl, gegebenenfalls durch Fluor, Chlor, Cyano, (C₁-C₄)-Alkyl, (C₁-C₄)-Alkoxy, (C₁-C₄)-Alkoxycarbonyl oder (C₃-C₆)-Cycloalkyl substituiertes (C₁-C₄)-Alkoxycarbonyl, gegebenenfalls durch Fluor, Chlor, Cyano, (C₁-C₄)-Alkyl, (C₁-C₄)-Alkoxy, (C₁-C₄)-Alkoxycarbonyl oder (C₃-C₆)-Cycloalkyl substituiertes (C₃-C₆)-Cycloalkyl und gegebenenfalls durch Fluor, Chlor, Cyano, (C₁-C₄)-Alkyl, (C₁-C₄)-Alkoxy, (C₁-C₄)-Alkoxycarbonyl oder (C₃-C₆)-Cycloalkyl substituiertes (C₁-C₄)-Alkoxy;
- R^{c}: insbesondere besonders bevorzugt ausgewählt ist aus der Gruppe, bestehend aus 1 '-Methyl, 2'-Methyl, 2'2'-Dimethyl, 2',3'-Dimethyl, 1',2'- Dimethyl, 1'-Cyano, 2'-Fluor, 2',2'-Difluor, 2',2'-Dichlor, 2'-Methoxycarbonyl, 2'-Ethoxycarbonyl und 1'-Cyano-2'methoxycarbonyl.

Von einer zweiten Ausführungsform der vorliegenden Erfindung werden Verbindungen der allgemeinen Formel (I) umfasst, in welchen
- R²: bevorzugt ausgewählt ist aus der Gruppe, bestehend aus gegebenenfalls durch Fluor, Chlor, Brom, Cyano, (C₁-C₄)-Alkyl, (C₁-C₄)-Alkoxy, (C₁-C₄)-Alkylcarbonyl, (C₁-C₄)-Alkoxycarbonyl oder (C₁-C₃)-Cycloalkyl substituiertes Alkyl mit 1 bis 4 Kohlenstoffatomen, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Alkenyl oder Alkinyl mit jeweils 2 bis 4 Kohlenstoffatomen, gegebenenfalls durch Fluor, Chlor, Brom, Cyano, (C₁-C₄)-Alkoxy, (C₁-C₄)-Alkyl-carbonyl oder (C₁-C₄)-Alkoxycarbonyl substituiertes Cycloalkyl mit 3 bis 6 Kohlenstoffatomen, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Cycloalkylalkyl mit 4 bis 9 Kohlenstoffatomen, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Phenyl oder Benzyl;
- R²: besonders bevorzugt ausgewählt ist aus der Gruppe, bestehend aus gegebenenfalls durch Fluor, Chlor, Brom, Cyano, (C₁-C₄)-Alkyl, (C₁-C₄)-Alkoxy, (C₁-C₄)-Alkyl-carbonyl, (C₁-C₄)-Alkoxy-carbonyl oder (C₁-C₃)-Cycloalkyl substituiertes Alkyl mit 1 bis 4 Kohlenstoffatomen, (C₂-C₄)-Alkenyl oder (C₂-C₄)-Alkinyl, (C₃-C₆)-Cycloalkyl, Phenyl oder Benzyl;
- R²: insbesondere besonders bevorzugt ausgewählt ist aus der Gruppe, bestehend aus Methyl, Ethyl, Propyl, Butyl, Isopropyl, Isobutyl, Cyclopropylmethyl, Propenyl, Propinyl, Chlorethyl, Benzyl oder Phenyl.

Von einer dritten Ausführungsform der vorliegenden Erfindung werden Verbindungen der allgemeinen Formel (I) umfasst, in welchen
- R³: bevorzugt ausgewählt ist aus der Gruppe, bestehend Wasserstoff, Hydroxy, Amino, Cyano, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes (C₁-C₄)-Alkyl, jeweils gegebenenfalls durch Fluor, Chlor, Brom, Cyano, (C₁-C₄)-Alkoxy oder (C₁-C₄)-Alkoxycarbonyl substituiertes (C₁-C₄)-Alkoxy, jeweils gegebenenfalls durch Fluor, Chlor, Brom, Cyano und/oder (C₁-C₄)-Alkyl substituiertes (C₃-C₆)-Cycloalkyl;
- R³: besonders bevorzugt ausgewählt ist aus der Gruppe, bestehend aus Wasserstoff, Hydroxy, Amino, Cyano, (C₁-C₄)-Alkyl, (C₁-C₄)-Alkoxy, (C₃-C₆)-Cycloalkyl;
- R³: insbesondere besonders bevorzugt ausgewählt ist aus der Gruppe, bestehend aus Methyl, Ethyl, Ethoxy, Cyclopropyl.

Von einer vierten Ausführungsform der vorliegenden Erfindung werden Verbindungen der allgemeinen Formel (I) umfasst, in welchen
- R⁴: bevorzugt ausgewählt ist aus der Gruppe, bestehend aus Wasserstoff, Hydroxy, Amino, Cyano, Fluor, Chlor, Brom, Iod, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Alkyl mit 1 bis 4 Kohlenstoffatomen, jeweils gegebenenfalls durch Fluor, Chlor und/oder Brom substituiertes Alkenyl oder Alkinyl mit jeweils 2 bis 4 Kohlenstoffatomen, jeweils gegebenenfalls durch Fluor, Chlor, Cyano, (C₁-C₄)-Alkoxy oder (C₁-C₄)-Alkoxycarbonyl substituiertes Alkoxy, Alkylthio, Alkylamino oder Alkylcarbonylamino mit jeweils 1 bis 4 Kohlenstoffatomen in der Alkylgruppe, Alkenyloxy, Alkinyloxy, Alkenylthio, Alkinylthio, Alkenylamino oder Alkinylamino mit jeweils 3 bis 4 Kohlenstoffatomen in der Alkenyl- oder Alkinylgruppe, jeweils gegebenenfalls durch Fluor, Chlor, Brom, Cyano und/oder (C₁-C₄)-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 4 Kohlenstoffatomen in der Cycloalkyl-bzw. Cycloalkenylgruppe und gegebenenfalls 1 bis 4 Kohlenstoffatomen im Alkylteil oder jeweils gegebenenfalls durch Fluor, Chlor, Brom, Cyano, Nitro, (C₁-C₄)-Alkyl, Trifluormethyl, (C₁-C₄)-Alkoxy und/oder (C₁-C₄)-Alkoxycarbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil;
- R⁴: besonders bevorzugt ausgewählt ist aus der Gruppe, bestehend aus Wasserstoff, Hydroxy, Amino, Cyano, Fluor, Chlor, Brom, Iod, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Alkyl mit 1 bis 4 Kohlenstoffatomen, jeweils gegebenenfalls durch Fluor, Chlor, Cyano, (C₁-C₄)-Alkoxy oder (C₁-C₄)-Alkoxycarbonyl substituiertes Alkoxy mit 1 bis 4 Kohlenstoffatomen in der Alkylgruppe, jeweils gegebenenfalls durch Fluor, Chlor, Cyano, (C₁-C₄)-Alkoxy oder (C₁-C₄)-Alkoxycarbonyl substituiertes Alkoxy, Alkylthio, mit jeweils 1 bis 4 Kohlenstoffatomen in der Alkylgruppejeweils, und gegebenenfalls durch Fluor, Chlor, Brom, Cyano und/oder (C₁-C₄)-Alkyl substituiertes Cycloalkyl, mit 3 bis 4 Kohlenstoffatomen in der Cycloalkylgruppe;
- R⁴: insbesondere besonders bevorzugt ausgewählt ist aus der Gruppe, bestehend aus Methyl, Ethyl, Propyl, Methoxy, Ethoxy, Propoxy, Isopropoxy, Trifluor-ethoxy, Methoxymethyl, Cyclopropyl, Isopropyl und Methoxymethyl.

Von einer fünften Ausführungsform der vorliegenden Erfindung werden Verbindungen der allgemeinen Formel (I) umfasst, in welchen
- n: bevorzugt 0, 1, 2 oder 3 bedeutet;
- n: besonders bevorzugt 0, 1 oder 2 bedeutet;
- n: insbesondere besonders bevorzugt 0 bedeutet.

Im Rahmen dieser Ausführungsformen der vorliegenden Erfindung ist es möglich, die einzelnen Allgemeinen, bevorzugten, besonders bevorzugten und insbesondere besonders bevorzugten Bedeutungen für die Substituenten Q, R¹, bzw. R^{c} und der Index n sowie R² bis R⁴ beliebig miteinander zu kombinieren. Das heißt, dass Verbindungen der allgemeinen Formel (I) von der vorliegenden Erfindung umfasst sind, in welchen beispielswiese der Substituent R^{c} eine bevorzugte Bedeutung aufweist und die Substituenten R² bis R⁴ und der Index n die allgemeine Bedeutung aufweisen oder aber beispielsweise der Substituent R² eine bevorzugte Bedeutung aufweist, der Substituent R³ eine besonders bevorzugte Bedeutung, der und die übrigen Substituenten und der Index n die allgemeine Bedeutung aufweisen. Diese einzelnen Kombinationen werden aus Übersichtsgründen nicht expressis verbis genannt, gelten aber im Rahmen der vorliegenden Erfindung als umfasst.

Zusätzlich zu den oben genannten Neutralverbindungen gemäß der allgemeinen, bevorzugten, besonders bevorzugten und insbesondere besonders bevorzugten Komponenten und im Rahmen allerer ihrer Kombinierbarkeiten werden in der vorliegenden Erfindung auch Salze der allgemeinen Formel (Ia) umfasst, 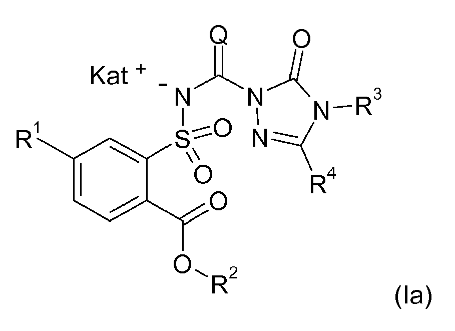 in welchen R¹, bzw. R^{c} und der Index n, R², R³, R⁴ und Q die zuvor genannten Bedeutungen haben und in welchen
- Kat ⁺: bevorzugt ausgewählt ist aus der Gruppe, bestehend aus einem Kation der Alkalimetalle, Erdalkalimetalle oder einem Ammonium-Ion, bei dem gegebenenfalls ein, zwei, drei oder alle vier Wasserstoffatome durch gleiche oder verschiedene Reste aus der Gruppe, bestehend aus gegebenenfalls substituierten (C₁-C₄)-Alkyl, Hydroxy-(C₁-C₄)-alkyl, (C₃-C₆)-Cycloalkyl, (C₁-C₄)-Alkyoxy-(C₁-C₄)-alkyl, Hydroxy-(C₁-C₄)-alkoxy-( C₁-C₄)-alkyl, Phenyl oder Benzyl substituiert sind,
- Kat ⁺: besonders bevorzugt ausgewählt ist aus der Gruppe, bestehend aus einem Ion der Alkalimetalle, einem Ammonium-Ion, bei dem gegebenenfalls ein, zwei, drei oder alle vier Wasserstoffatome durch gleiche oder verschiedene Reste aus der Gruppe, bestehend aus gegebenenfalls substituierten (C₁-C₄)-Alkyl, Hydroxy-( C₁-C₄)-alkyl, (C₃-C₆)-Cycloalkyl, (C₁-C₄)-Alkyoxy-(C₁-C₄)-alkyl, oder Hydroxy-(C₁-C₄)-alkoxy-( C₁-C₄)-alkyl, substituiert sind,
- Kat⁺: insbesondere besonders bevorzugt ausgewählt ist aus der Gruppe, bestehend aus Na⁺, K⁺, NH₄⁺, Bu₄N⁺, (HO(CH₂-CH₂))₃NH⁺.

In einer weiteren Ausgestaltung der vorliegenden Erfindung weist die Verbindung der allgemeinen Formel (I) die folgende Struktur (Ib) auf, in welcher R² Methyl bedeutet:

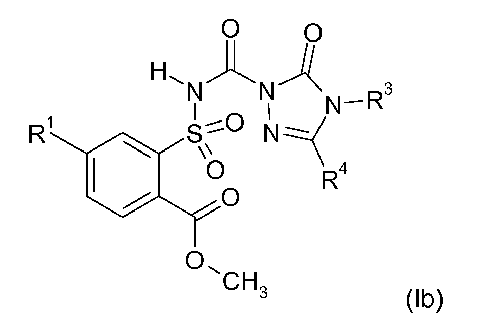

Die übrigen Substituenten R¹, bzw R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ib) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Ic) auf, in welcher R² Ethyl bedeutet:

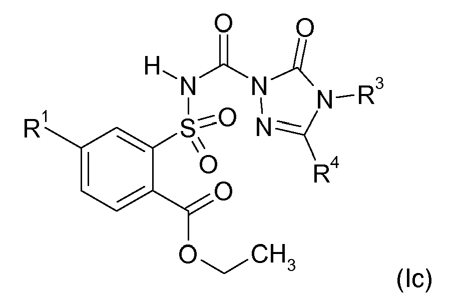

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ic) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Id) auf, in welcher R² Propyl bedeutet:

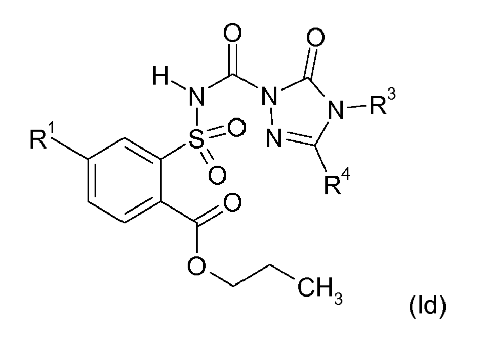

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Id) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Ie) auf, in welcher R² Isopropyl bedeutet:

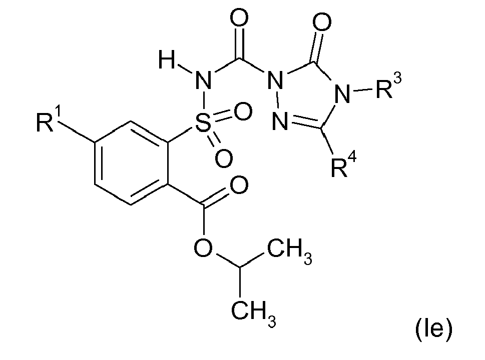

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ie) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (If) auf, in welcher R² Butyl bedeutet:

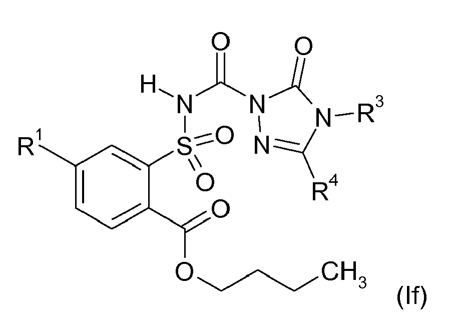

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ie) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Ig) auf, in welcher R² Isobutyl bedeutet:

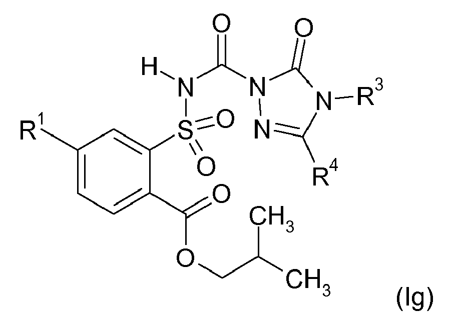

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ig) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Ih) auf, in welcher R² Cyclopropylmethyl bedeutet:

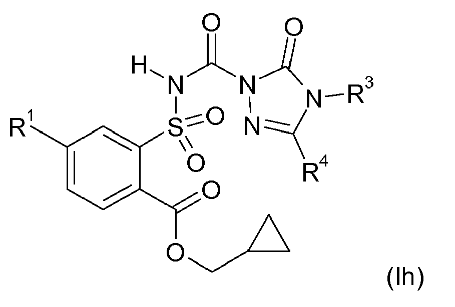

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ih) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Ii) auf, in welcher R² Allyl bedeutet:

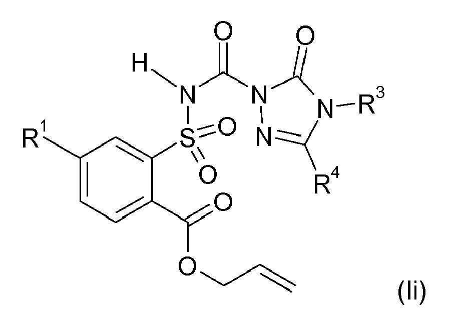

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ii) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Ij) auf, in welcher R² Propargyl bedeutet:

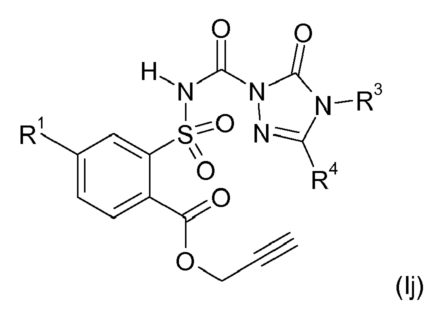

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ij) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Ik) auf, in welcher R² 2-Chlorethyl bedeutet:

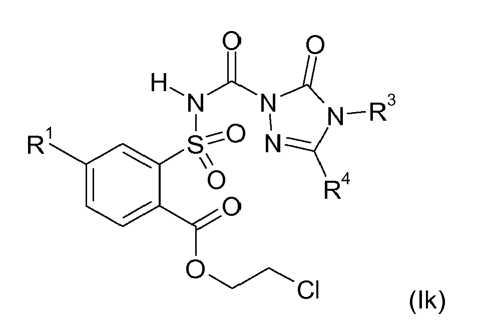

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Ik) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (Im) auf, in welcher R² Benzyl bedeutet:

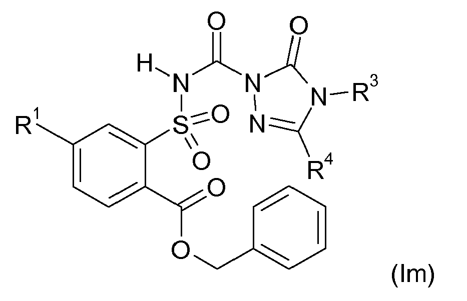

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (Im) als Salze gemäß der Definition der Verbindungen der allgemeinen Formel (Ia) vorliegen.

In einer weiteren Ausgestaltung der vorliegenden Erfindung weist die Verbindung der allgemeinen Formel (I) die folgende Struktur (In) auf, in welcher R² Phenyl bedeutet:

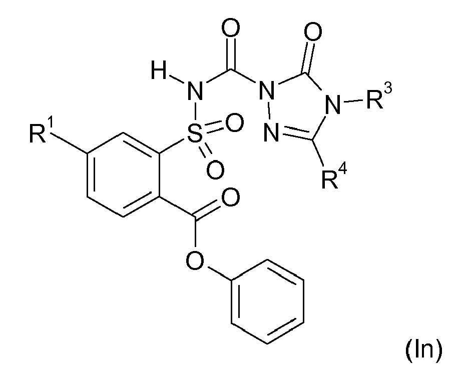

Die übrigen Substituenten R¹, bzw. R^{c} und der Index n, sowie R³ und R⁴ weisen die oben für die Verbindungen der allgemeinen Formel (I) im Allgemeinen, im bevorzugten, im besonders bevorzugten und im insbesondere besonders bevorzugten definierten Bedeutungen auf. Ebenso können die Verbindungen der allgemeinen Formel (In) als Salze gemäß der Definition der Verbindungen der allgemeinen Formel (Ia) vorliegen.

In den Verbindungen der allgemeinen Formel (I) und/oder deren Salze gemäß der allgemeinen Formel (Ia) weisen die Substituenten und Reste R¹, bzw. Rc und der Index n, sowie R², R³, R⁴, Q und Kat⁺ die vorstehend allgemeinen, bevorzugten, besonders bevorzugten und insbesondere besonders bevorzugten Bedeutungen auf.

In der allgemeinen Formel (I) und/oder deren Salze gemäß der allgemeinen Formel (Ia) und allen übrigen Formeln in der vorliegenden Erfindung können die Reste Alkyl, Alkoxy, Haloalkyl, Alkoxyalkyl, Alkenyl, Alkinyl, Haloalkoxy, Alkylamino, Dialkylamino, Alkylthio und Haloalkylthio, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, Propyle wie n-Propyl oder Isopropyl, Butyle wie n-Butyl, Isobutyl oder tert-Butyl, Pentyle wie n-Pentyl, Isopentyl oder neoPentyl, Hexyle wie n-Hexyl, Isohexyl, 3-Methylpentyl, 2,2-Dimethylbutyl oder 2,3-Dimethylbutyl, Heptyle, wie n-Heptyl, 1-Methylhexy oder 1,4-Dimethylpentyl; Alkenylund Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tricyclischer Form vorkommen. Cycloalkylalkyl-Gruppen haben die Bedeutungen, welche sich durch Kombination von Cycloalkylgruppen mit Alkylgruppen ergeben.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl. Der Begriff "Halo" wird an dieser und weiteren Stellen erfindungsgemäß synonym zu "Halogen" verwendet.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen (sofern nicht andersweitig definiert), sowie die entsprechenden ungesättigten und/oder substituierten Reste im Kohlenstoffgerüst, die jeweils geradkettig oder verzweigt sein können. Beispiele sind Methylen, Ethylen, n-Propylen und Isopropylen und n-Butylen, sec-Butylen, Isobutylen, tert-Butylen.

Hydroxyalkylgruppen als gegebenenfalls substituierte Alkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder Iod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl, Perhaloalkyl, CF₃, CHF₂, CH₂F, CF₃CF₂, CH₂FCHCl, CCl₃, CHCl₂, CH₂CH₂Cl, Haloalkoxy ist z.B. OCF₃, OCHF₂, OCH₂F, CF₃CF₂O, OCH₂CF₃ und OCH₂CH₂Cl, entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl. Aralkyl ist mit Alkyl substituiertes Aryl, wobei Alkyl und Aryl jeweils die angegebenen Definitionen aufweisen. Mit der Definition "mit einem oder mehreren Resten substituiert ist" sind, wenn nicht anders definiert, ein oder mehrere gleiche oder verschiedene Reste gemeint.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bei Resten mit C-Atomen sind solche mit 1 bis 6 C-Atomen, vorzugsweise 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)-Alkyl, vorzugsweise Methyl oder Ethyl, (C₁-C₄)-Haloalkyl, vorzugsweise Trifluormethyl, (C₁-C₄)-Alkoxy, vorzugsweise Methoxy oder Ethoxy, (C₁-C₄)-Haloalkoxy, Hydroxy, Nitro und Cyano.

Wenn ein Arylrest substituiert ist, so kann es sich vorzugsweise um Phenyl, das ein oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)- Halogenalkyl, (C₁-C₄)-Halogenalkoxy, Cyano und Nitro substituiert ist, z.B. ortho-, meta- und para-Tolyl, Dimethylphenyle, 2-, 3- und 4-Chlorphenyl, 2-, 3- und 4-Trifluormethyl und 2-, 3- und 4-Trichlormethyl-phenyl, 2,4-, 3,5-, 2,5- und 2,3-Dichlorphenyl, ortho-, meta- und para-Methoxyphenyl.

Die vorliegenden Verbindungen der allgemeinen Formel (I) und/oder deren Salze gemäß der allgemeinen Formel (Ia) können gegebenenfalls mindestens ein chirales Kohlenstoffatom aufweisen. Gemäß den Regeln nach Cahn, Ingold und Prelog (CIP-Regeln) können diese Kohlenstoffatome sowohl eine (R)- als auch eine (S)-Konfiguration aufweisen.
Von der vorliegenden Erfindung werden Verbindungen der allgemeinen Formel (I) und/oder deren Salze gemäß der allgemeinen Formel (Ia) sowohl mit (S)- als auch mit (R)-Konfiguration an den jeweiligen chiralen Kohlenstoffatomen umfasst, d.h., dass die vorliegende Erfindung die Verbindungen der allgemeinen Formel (I) und/oder deren Salze gemäß der allgemeinen Formel (Ia) erfasst, in welchen die betreffenden Kohlenstoffatome jeweils unabhängig voneinander
(1) eine (R)-Konfiguration; oder
(2) eine (S)-Konfiguration
aufweisen. Wenn mehrere Chiralitätszentren in den Verbindungen der allgemeinen Formel (I) und/oder deren Salzen gemäß der allgemeinen Formel (Ia) vorliegen, sind beliebige Kombinationen der Konfigurationen der Chiralitätszentren möglich, d.h. dass
(1) ein Chiralitätszentrum die (R)-Konfiguration und das andere Chiralitätszentrum die (S)-Konfiguration;
(2) ein Chiralitätszentrum die (R)-Konfiguration und das andere Chiralitätszentrum die (R)-Konfiguration; und
(3) ein Chiralitätszentrum die (S)-Konfiguration und das andere Chiralitätszentrum die (S)-Konfiguration aufweist.

Die durch ihre spezifische Raumform definierten möglichen Stereoisomeren, wie Enantiomere, Diastereomere und Z- und E-Isomere, sind im Rahmen der vorliegenden Erfindung vollständig von der Definition der allgemeinen Formel (I) und Formel (Ia) umfaßt, d.h. dass sowohl die reinen Stereoisomere als auch weniger reine Mischungen davon von der vorliegenden Erfindung erfasst sind.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.

Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Aus-gangsund/oder Hilfsstoffe selektiv hergestellt werden. Die Erfindung betrifft somit auch alle Stereoisomeren, die von der allgemeinen Formel (I) und Formel (Ia) umfasst werden, jedoch nicht mit ihrer spezifischen Stereoform angegeben sind und deren Gemische.

Die Kombinationsmöglichkeiten der verschiedenen Substituenten der allgemeinen Formel (I) und Formel (Ia) sind so zu verstehen, dass die allgemeinen Grundsätze des Aufbaus chemischer Verbindungen zu beachten sind, d.h. die Formel (I) und/oder Formel (Ia) nicht Verbindungen umfasst, von denen der Fachmann weiß, dass sie chemisch nicht möglich sind.

Herstellung der erfindungsgemäßen Verbindungen der allgemeinen Formel (I) und/oder deren Salze gemäß der allgemeinen Formel (Ia).

Weiterer Gegenstand der vorliegenden Erfindung sind Verfahren zur Herstellung entsprechender Verbindungen der allgemeinen Formel (I) und/oder deren Salze gemäß der allgemeinen Formel (Ia).

In einer ersten Ausführungsform der vorliegenden Erfindung erfolgt die Herstellung der Verbindungen der allgemeinen Formel (I) dadurch, dass man gegebenenfalls substituierte 4-Cyclopropyl-substituierte Phenylsulfonamide der allgemeinen Formel (II), 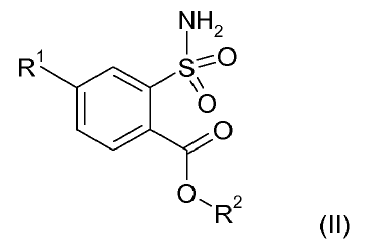 wobei die übrigen Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen,
mit einem heterocyclischen (Thio)Carbonsäurederivat der Formel (III), 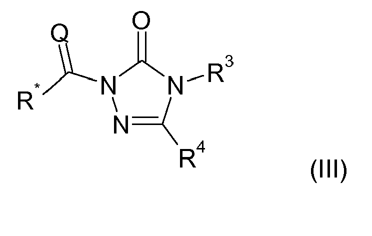 wobei die Substituenten Q, R³ und R⁴ die oben für die Verbindungen der allgemeinen Formel (I) im Allgemeinen, im bevorzugten, im besonders bevorzugten und im insbesondere besonders bevorzugten definierten Bedeutungen aufweisen, gegebenenfalls in Gegenwart eines Reaktionshilfsmittels, insbesondere Säureakzeptors und gegebenenfalls in Gegenwart eines Verdünnungsmittels umsetzt,
wobei
- R*: für Halogen oder einen unsubstituierten oder substitierten (C₁-C₂₀)-Kohlenwasserstoffoxyrest wie unsubstituiertes oder substituiertes Alkoxy, Aryloxy, Arylalkoxy oder Alkylaryloxy steht, wobei bevorzugte Substituenten für die vier letztgenannten Reste Halogen, (C₁-C₄)-Alkyl, (C₁-C₄)-Alkyloxy und (C₁-C₄)-Halogenalkyl sind, vorzugsweise steht R* für Fluor, Chlor, Brom, (C₁-C₆)-Alkoxy, (C₆-C₁₀)-Aryloxy, (C₆-C₁₀)-Aryl-(C₁-C₆)-alkoxy oder (C₁-C₆)-Alkyl-(C₆-C₁₀)-aryloxy steht.

Die Verbindungen der allgemeinen Formel (II) sind im Stand der Technik noch nicht bekannt und sind somit ebenfalls ein weiterer Gegenstand der vorliegenden Erfindung.

Die Umsetzung der Verbindungen der Formeln (II) und (III) gemäß Variante a) erfolgt vorzugsweise basenkatalysiert in einem inerten organischen Lösungsmittel, wie z.B. Dichlormethan, Acetonitril, Dioxan oder Tetrahydrofuran (THF) bei Temperaturen zwischen 0 °C und dem Siedepunkt des Lösungsmittels, vorzugsweise bei Raumtemperatur (vgl. DE 19621685). Als Base werden dabei beispielsweise organische Aminbasen, wie 1,8-Diazabicyclo[5.4.0]undec-7-en (DBU), Alkali-tert-butoxide, wie z.B. Natrium-tert-Butylat, oder Alkalihydroxide, wie z.B. Natriumhydroxid, insbesondere bei R* = (subst.) Aryloxy (vgl. EP 44 807), oder Trialkylaluminium wie Trimethylaluminium oder Triethylaluminium, letztere insbesondere bei R* = Alkyloxy (vgl. EP 166 516) verwendet. Die jeweilige Base wird dabei beispielsweise im Bereich von 1 bis 3 Moläquivalenten, bezogen auf die Verbindung der Formel (II) eingesetzt.

Die Verbindungen der allgemeinen Formel (II) können dabei durch Umsetzung der Verbindungen der allgemeinen Formel (IV) mit einer starken Säure erhalten werden. Als starke Säuren kommen z.B. Mineralsäuren wie Schwefelsäure H₂SO₄ oder Salzsäure HCl oder starke organische Säuren wie Trifluoressigsäure in Frage. Die Reaktion erfolgt beispielsweise bei Temperaturen von -20°C bis zur jeweiligen Rückflusstemperatur des Reaktionsgemisches, vorzugsweise 0°C bis 40°C. Die Reaktionszeiten liegen zwischen 30 min und 2 Tagen. Die Umsetzung kann in Substanz oder auch in einem inerten Solvens, wie z.B. Dichlormethan oder Trichlormethan, durchgeführt werden (Schema 1), 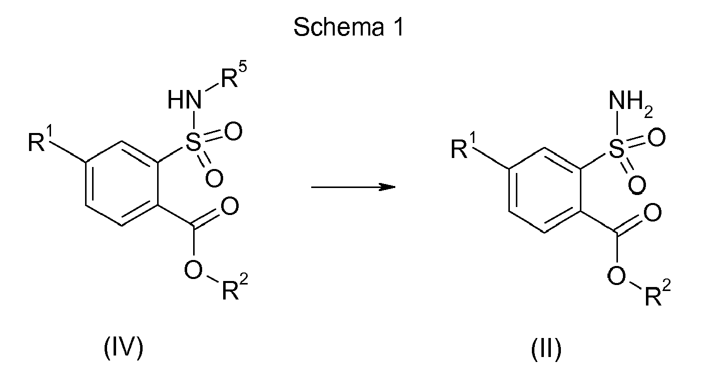 wobei die Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen und R⁵ eine verzweigte (C₁-C₈)-Alkyl-Gruppe, vorzugsweise eine verzweigte (C₁-C₄)-Alkyl-Gruppe, insbesondere bevorzugt eine tert-Butyl-Gruppe bedeutet.

Die Verbindungen der allgemeinen Formel (II) können ebenso durch Umsetzung der Verbindungen der allgemeinen Formel (V) mit gasförmigem Chlorwasserstoff in Gegenwart eines Alkohols R²-OH hergestellt werden. Man führt die Reaktion beispielsweise bei Temperaturen zwischen 0°C und dem Siedepunkt des Alkohols R²-OH durch, wobei der der Alkohol R²-OH gleichzeitig als Lösungsmittel dienen kann (DE 19748470, US 4566898) (Schema 2), 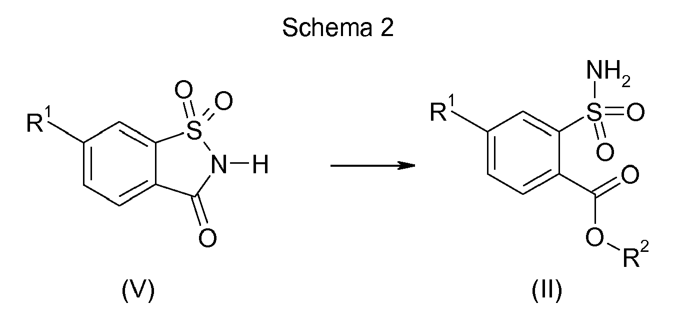 wobei die Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen.

Analog hierzu können Verbindungen der allgemeinen Formel (IV) aus Verbindungen der allgemeinen Formel (VI) durch basenkatalysierte Öffnung des Saccharinringes erhalten werden. Hierzu kann der jeweilige Alkohol R²-OH als Lösungsmittel dienen und als Base das entsprechende Alkoholat, bevorzugt das Natriumalkoholat, verwendet werden. Die Reaktion wird vorzugsweise bei Temperaturen zwischen - 20°C und der Siedetemperatur des Alkohols durchgeführt. (Schema 3), 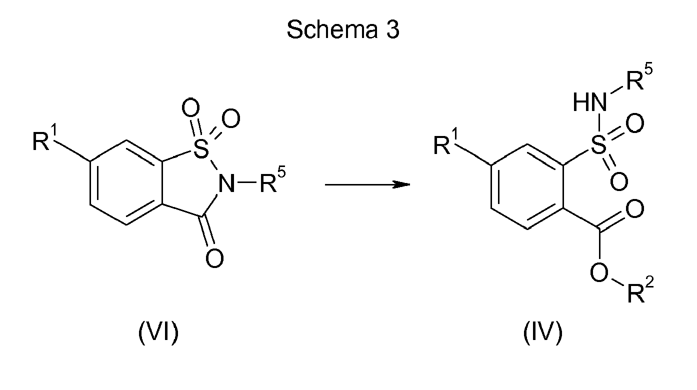 wobei die Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen und R⁵ eine verzweigte (C₁-C₈)-Alkyl-Gruppe, vorzugsweise eine verzweigte (C₁-C₄)-Alkyl-Gruppe, insbesondere bevorzugt eine tert-Butyl-Gruppe bedeutet.

Die Verbindungen der allgemeinen Formel (VI) können durch Umsetzung der Verbindungen der allgemeinen Formel (VII) mit gegebenenfalls substituierter Cyclopropylboronsäure oder einem gegebenenfalls substituierten Cyclopropylboronsäureester unter Suzuki-Bedingungen mit einer katalytischen Menge eines Übergangsmetall-Katalysators, vorzugsweise eines PalladiumKatalysators- wie z.B. Palladium(II)acetat und einer Ligand-Verbindung, vorzugsweise Trialkylphosphin oder Triarylphosphin, wie z.B. Tricyclohexylphosphin und einer Base, vorzugsweise Kalium-tert-Butylat, Trialkylamine oder Kalium- oder Caesiumcarbonat oder Kaliumphosphat in einem inerten Lösungsmittel (z. B. Toluol oder Dimethylformamid ggf. jeweils im Gemisch mit Wasser (3:1)) erhalten werden bei Reaktionstemperaturen zwischen -20°C und der Rückflusstemperatur des Lösemittels (Schema 4), 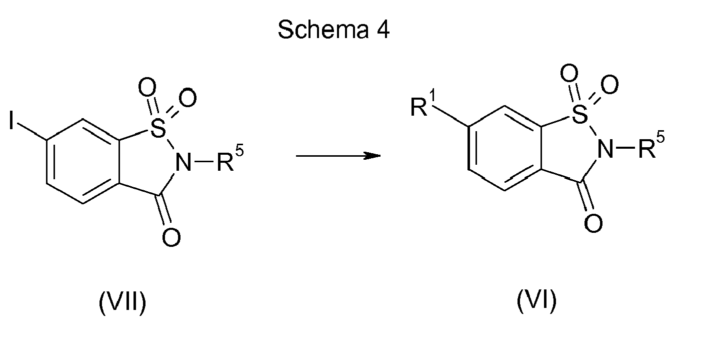 wobei der Substituent R¹, bzw. R^{c} und der Index n, die oben für die Verbindungen der allgemeinen Formel (I) im Allgemeinen, im bevorzugten, im besonders bevorzugten und im insbesondere besonders bevorzugten definierten Bedeutungen aufweist und R⁵ eine verzweigte (C₁-C₈)-Alkyl-Gruppe, vorzugsweise eine verzweigte (C₁-C₄)-Alkyl-Gruppe, insbesondere bevorzugt eine tert-Butyl-Gruppe bedeutet.

Mittels der eben beschriebenen Reaktionsbedingungen lässt sich ebenso die Cyclopropyl-Verbindung (V) aus der Iod-Verbindung (VIII) synthetisieren (Schema 5), 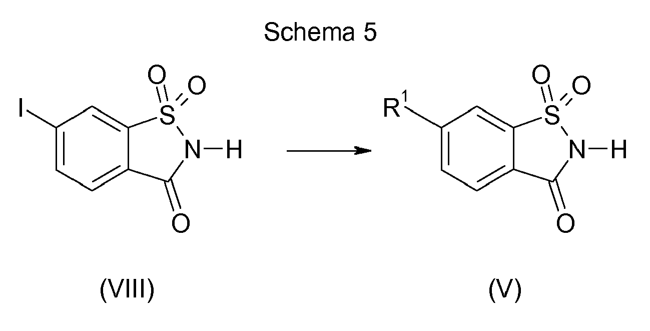 wobei der Substituent R¹, bzw R^{c} und der Index n, die oben für die Verbindungen der allgemeinen Formel (I) im Allgemeinen, im bevorzugten, im besonders bevorzugten und im insbesondere besonders bevorzugten definierten Bedeutungen aufweist. Substituierte Cyclopropanboronsäuren bzw. deren Ester lassen sich nach literaturbekannten Methoden herstellen (z.B. WO 2008/154221; WO 2008/147547; J. Org. Chem. 1966, 31, 2765; Synthesis 1991, 8, 605).

Zu den Verbindungen der allgemeinen Formel (VII) gelangt man zum Beispiel durch Verseifung der Verbindungen der allgemeinen Formel (IX) und nachfolgender Cyclisierung (siehe hierzu z.B. DE 19748470, WO96/05182, CN 1702064) (Schema 6), 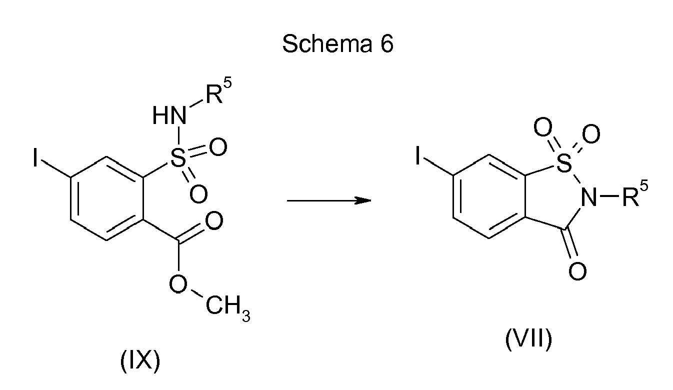 wobei der Substituent R⁵ eine verzweigte (C₁-C₈)-Alkyl-Gruppe, vorzugsweise eine verzweigte (C₁-C₄)-Alkyl-Gruppe, insbesondere bevorzugt eine tert-Butyl-Gruppe bedeutet.

Die Verbindungen mit der allgemeinen Formel (IX) können durch Umsetzung der Verbindungen mit der allgemeinen Formel (X) mit Alkoholen, z.B. Methanol zu den Verbindungen mit der allgemeinen Formel (XI) und anschließender Umsetzung mit Aminen, wie z.B. R⁵-NH₂ analog literaturbekannter Methoden erhalten werden (siehe hierzu z.B. WO 03/091228) (Schema 7), 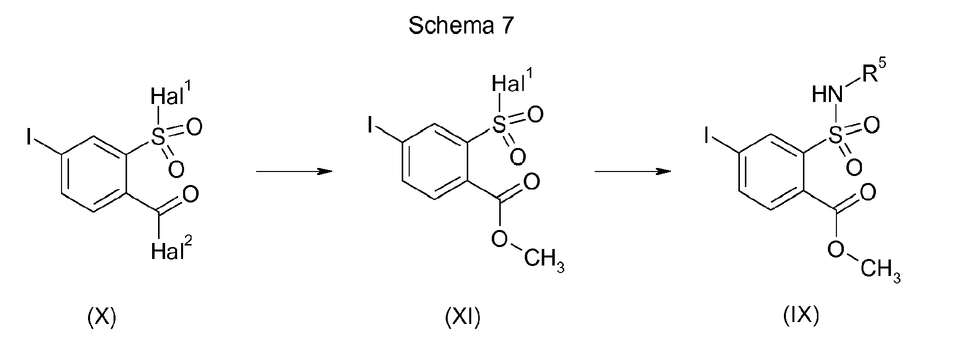 wobei R⁵ eine verzweigte (C₁-C₈)-Alkyl-Gruppe, vorzugsweise eine verzweigte (C₁-C₄)-Alkyl-Gruppe, insbesondere bevorzugt eine tert-Butyl-Gruppe bedeutet. Hal¹ und Hal² bedeuten unabhängig voneinander ein Halogenatom, vorzugsweise ein Chlor- oder Bromatom, insbesondere ein Chloratom.

Die Verbindungen mit der allgemeinen Formel (VI) können auch synthetisiert werden, indem man Verbindungen der allgemeinen Formel (IX) mit gegebenenfalls substituierter Cyclopropylboronsäure oder einem gegebenenfalls substituierten Cyclopropylboronsäureester unter Suzuki-Bedingungen (wie für Schema 4 beschrieben) zu den Verbindungen mit der allgemeinen Formel (IVa) umsetzt und danach analog Schema 5 verseift und cyclisiert (Schema 8). Die Verbindungen der allgemeinen Formel (VI) können unter den eben beschriebenen Reaktionsbedingungen für die Herstellung der Verbindungen der allgemeinen Formel (IVa) je nach Reaktionszeit und -temperatur auch direkt entstehen.

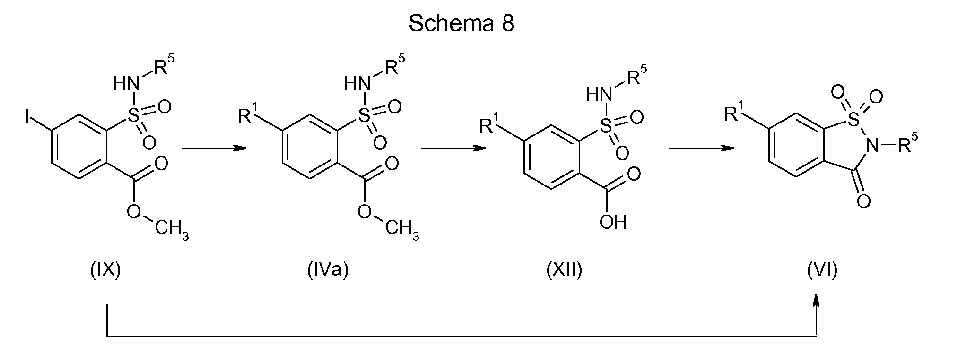

Der Substituent R¹, bzw. R^{c} und der Index n, weist die oben für die Verbindungen der allgemeinen Formel (I) im Allgemeinen, im bevorzugten, im besonders bevorzugten und im insbesondere besonders bevorzugten definierten Bedeutungen auf und R⁵ bedeutet eine verzweigte (C₁-C₈)-Alkyl-Gruppe, vorzugsweise eine verzweigte (C₁-C₄)-Alkyl-Gruppe, insbesondere bevorzugt eine tert-Butyl-Gruppe.

Die Verbindung (V) kann durch Umsetzung der Verbindungen der allgemeinen Formel (VI) mit einer starken Säure erhalten werden (analog Schema 1) (Schema 9), 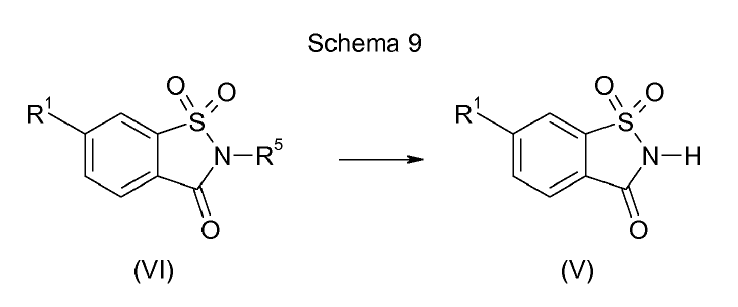 wobei der Substituent R¹, bzw. R^{c} und der Index n, die oben für die Verbindungen der allgemeinen Formel (I) im Allgemeinen, im bevorzugten, im besonders bevorzugten und im insbesondere besonders bevorzugten definierten Bedeutungen aufweist und R⁵ eine verzweigte (C₁-C₈)-Alkyl-Gruppe, vorzugsweise eine verzweigte (C₁-C₄)-Alkyl-Gruppe, insbesondere bevorzugt eine tert-Butyl-Gruppe bedeutet.

In einer zweiten Ausführungsform der vorliegenden Erfindung erfolgt die Herstellung der Verbindungen der allgemeinen Formel (I) dadurch, dass man gegebenenfalls substituierte 4-Cyclopropyl-substituierte Phenylsulfonsäureiso(thio)cyanate der allgemeinen Formel (XIII), 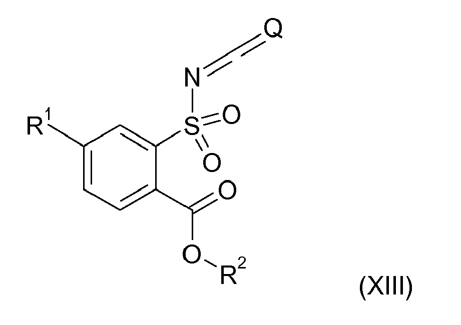 wobei die Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen,
mit einer Heterocyclylverbindung der Formel (XIV), 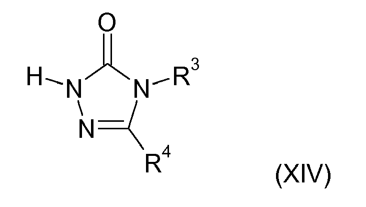 wobei die Substituenten R³ und R⁴ die oben für die Verbindungen der allgemeinen Formel (I) im Allgemeinen, im bevorzugten, im besonders bevorzugten und im insbesondere besonders bevorzugten definierten Bedeutungen aufweisen,
gegebenenfalls in Gegenwart eines Reaktionshilfsmittels und gegebenenfalls in Gegenwart eines Verdünnungsmittels umsetzt.

In einer dritten Ausführungsform der vorliegenden Erfindung erfolgt die Herstellung der Verbindungen der allgemeinen Formel (I) dadurch, dass man gegebenenfalls substituierte 4-Cyclopropyl-substituierte Phenylsulfonylhalogenide der allgemeinen Formel (XV), 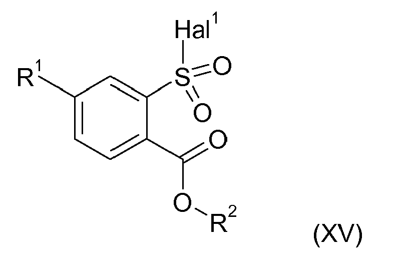 wobei die Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen und Hal¹ ein Halogenatom, vorzugsweise ein Chlor- oder Bromatom, insbesondere ein Chloratom bedeutet,
mit einer Heterocyclylverbindung der Formel (XIV), 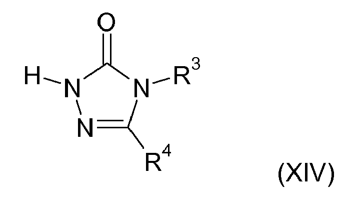 wobei die Substituenten R³ und R⁴ die oben für die Verbindungen der allgemeinen Formel (I) im Allgemeinen, im bevorzugten, im besonders bevorzugten und im insbesondere besonders bevorzugten definierten Bedeutungen aufweisen,
und einem Metall(thio)cyanat der Formel (XVI)

MQCN (XVI)

wobei
- M: ein Kation, z.B. ein Ammoniumkation oder Alkalimetallkation, vorzugsweise ein Natrium- oder Kaliumion ist und
- Q: ein Sauerstoffatom oder ein Schwefelatom ist,
gegebenenfalls in Gegenwart eines Reaktionshilfsmittels und gegebenenfalls in Gegenwart eines Verdünnungsmittels umsetzt.

In einer vierten Ausführungsform der vorliegenden Erfindung erfolgt die Herstellung der Verbindungen der allgemeinen Formel (I) dadurch, dass man gegebenenfalls substituiertes 4-Cyclopropyl-substituierte Phenylsulfonylhalogenide der allgemeinen Formel (XV), 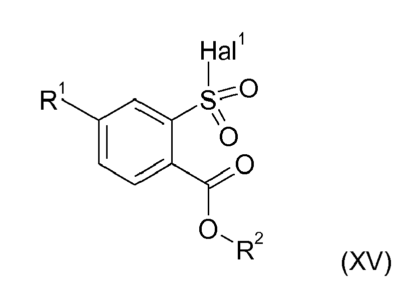 wobei die Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen, und Hal¹ ein Halogenatom, vorzugsweise ein Chlor- oder Bromatom, insbesondere ein Chloratom ist,
mit einem (Thio)Carbonsäureamid der Formel (XVII), 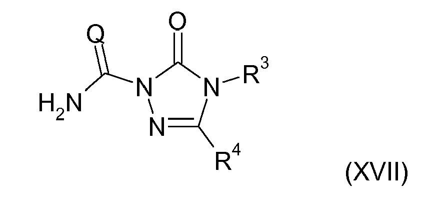 wobei die Substituenten R³, R⁴ und Q die oben für die Verbindungen der allgemeinen Formel (I) im Allgemeinen, im bevorzugten, im besonders bevorzugten und im insbesondere besonders bevorzugten definierten Bedeutungen aufweisen, gegebenenfalls in Gegenwart eines Reaktionshilfsmittels, insbesondere eines Säureakzeptors und gegebenenfalls in Gegenwart eines Verdünnungsmittels umsetzt.

In einer fünften Ausführungsform der vorliegenden Erfindung erfolgt die Herstellung der Verbindungen der allgemeinen Formel (I) dadurch, dass man gegebenenfalls substituierte 4-Cyclopropyl-substituierte Phenylsulfonylamino(thio)carbonylverbindung der allgemeinen Formel (XVIII), 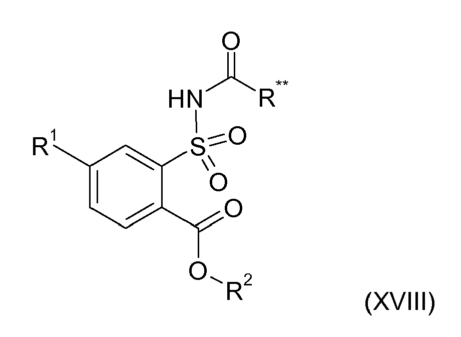 wobei die Substituenten R¹, bzw. R^{c} und der Index n, und R² die oben für die Verbindungen der allgemeinen Formel (I) im Allgemeinen, im bevorzugten, im besonders bevorzugten und im insbesondere besonders bevorzugten definierten Bedeutungen aufweisen, und
- R**: für Halogen oder einen unsubstituierten oder substitierten (C₁-C₂₀)-Kohlenwasserstoffoxyrest wie unsubstituiertes oder substituiertes Alkoxy, Aryloxy, Arylalkoxy oder Alkylaryloxy steht, wobei bevorzugte Substituenten für die vier letztgenannten Reste Halogen, (C₁-C₄)-Alkyl, (C₁-C₄)-Alkyloxy und (C₁-C₄)-Halogenalkyl sind; vorzugsweise steht R** für Fluor, Chlor, Brom, (C₁-C₆)-Alkoxy, (C₆-C₁₀)-Aryloxy, (C₆-C₁₀)Aryl-, (C₁-C₆)-Alkoxy- oder (C₁-C₆)-Alkyl-(C₆-C₁₀)aryloxy,
mit einer Heterocyclylverbindung der Formel (XIV), 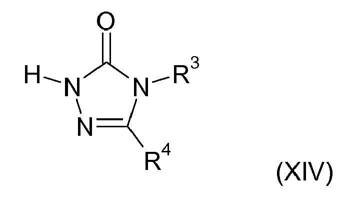 wobei die Substituenten R³ und R⁴ die oben für die Verbindungen der allgemeinen Formel (I) im Allgemeinen, im bevorzugten, im besonders bevorzugten und im insbesondere besonders bevorzugten definierten Bedeutungen aufweisen, gegebenenfalls in Gegenwart eines Reaktionshilfsmittels, insbesondere eines Säureakzeptors und gegebenenfalls in Gegenwart eines Verdünnungsmittels umsetzt.

Die Salze der 4-Cyclopropyl-substituierten Phenylsulfonylamino(thio)carbonyltriazolinone der allgemeinen Formel (Ia) lassen sich aus der Neutralform der Phenylsulfonylamino(thio)carbonyl-triazolinone oder aus Salzen der Phenylsulfonylamino(thio)carbonyltriazolinone, insbesondere Alkalimetallsalzen (siehe z.B. EP-A-30138, EP-A-7687) oder auch ausgehend von Salzen von gegebenenfalls substituierten 4-Cyclopropyl-substituierten Phenylsulfonamiden der allgemeinen Formel (II) z.B. auf folgende Weise herstellen:

Deprotonierung der neutralen 4-Cyclopropyl-substituierten Phenylsulfonylamino-(thio)carbonyltriazolinone der allgemeinen Formel (I) mit einer geeigneten Base der Formel Kat ⁺ B - (Schema 10), wobei B ⁻ zum Beispiel Hydrid, Hydroxy- oder Alkoxyanionen, wie Methoxy, Ethoxy, n-Propoxy, Isopropoxy, n-Butoxy oder tert-Butoxy darstellen, 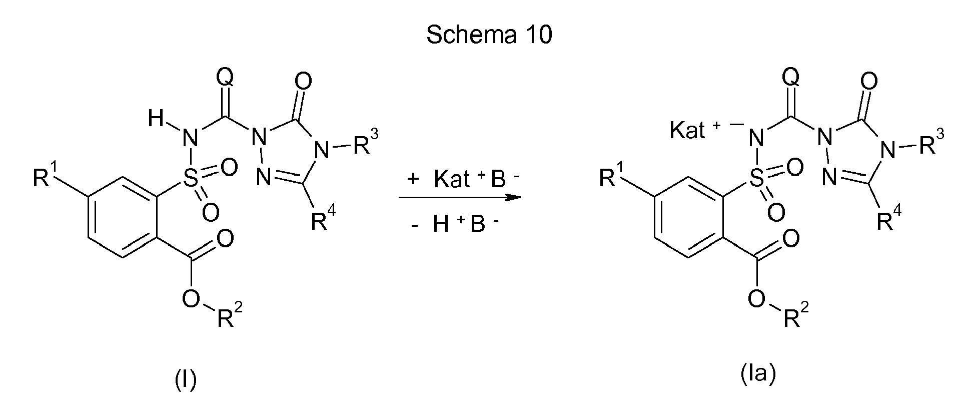 wobei die Substituenten R¹, bzw. R^{c} und der Index n, sowie R², R³, R⁴, Q und Kat ⁺ die oben für die Verbindungen der allgemeinen Formel (I) und/oder deren Salze gemäß der allgemeinen Formel (Ia) im Allgemeinen, im bevorzugten, im besonders bevorzugten und im insbesondere besonders bevorzugten definierten Bedeutungen aufweisen.

Hierzu werden die 4-Cyclopropyl-substituierten Phenylsulfonylamino(thio)carbonyltriazolinone der Formel (I) in einem inerten Lösungsmittel oder Lösungsmittelgemisch gelöst oder suspendiert und mit einem Äquivalent an Kat⁺ B ⁻ bei Temperaturen zwischen -20°C uns 100°C, vorzugsweise zwischen -10°C und 50°C umgesetzt.

Neben den erfindungsgemäßen Verbindungen der allgemeinen Formel (I) und/oder deren Salze gemäß der allgemeinen Formel (Ia) und den speziellen Ausführungen der allgemeinen Formel (Ib), (Ic), (Id), (Ie), (If), (Ig), (Ih), (Ii), (Ij), (Ik), (Im), (In) sind auch die Sulfonamide der allgemeinen Formel (II) und die weiteren Intermediate der allgemeinen Formel (IV), bzw. in der speziellen Ausführung mit der allgemeinen Formel (IVa) und die weiteren Intermediate der allgemeinen Formel (VI), (XII), (XIII), (XV), (XVIII) wie auch die Verbindung (V) neue Verbindungen, die ebenso wie ihre Herstellung und ihre Verwendung zur Herstellung von Verbindungen der allgemeinen Formel (I) und Formel (Ia) Gegenstand der vorliegenden Erfindung sind.

### A. Synthesebeispiele

### 1. 4-Cyclopropyl-2-{[(4-cyclopropyl-5-oxo-3-propoxy-4,5-d ihydro-1H-1,2,4-triazol-1-yl)carbonyl]sulfamoyl}benzoesäuremethylester (Verbindung I-55)

195 mg (0.76 mmol) 4-Cyclopropyl-2-sulfamoylbenzoesäuremethylester wurden in 2 ml Acetonitril vorgelegt und mit 0.26 ml (1.76 mmol) DBU (1,8-Diazobicyclo[5.4.0]undec-7-en) und danach mit 300 mg (1 mmol) 4-Cyclopropyl-5-oxo-1-(N)phenoxycarbonyl-3-propoxy-4,5-dihydro-1H-1,2,4-triazol versetzt. Die Reaktionsmischung wurde 1h bei Raumtemperatur gerührt. Danach wurden 20 ml 1N Salzsäure zugegeben. Die flüssigen Phasen über dem entstandenen Öl wurden dekantiert. Der Rückstand wurde mehrmals mit Wasser gewaschen und danach in Dichlormethan aufgenommen, die organische Phase getrocknet (Magnesium) und ins Trockne eingedampft. Das Rohprodukt wurde mittels präparativer HPLC gereinigt. Man erhielt 60 mg (16% d. Th.) 4-Cyclopropyl-2-{[(4-cyclopropyl-5-oxo-3-propoxy-4,5-dihydro-1H-1,2,4-triazol-1 - yl)carbonyl]sulfamoyl}benzoesäuremethylester (Verbindung I-55).

### 2. 4-Cyclopropyl-2-{[(4-cyclopropyl-3-methoxy-5-oxo-4,5-dihydro-1H-1,2,4-triazol-1-yl)carbonyl]sulfamoyl}benzoesäuremethylester (Verbindung I-39)

51.9 g (203 mmol) 4-Cyclopropyl-2-sulfamoylbenzoesäuremethylester (Verbindung II-1) und 55.9 g (203 mmol) 4-Cyclopropyl-3-methoxy-5-oxo-1-(N)phenoxycarbonyl-4,5-dihydro-1H-1,2,4-triazol wurden in 1.5 I Acetonitril vorgelegt und bei 0°C langsam 60.7 ml (406 mmol) DBU zugetropft. Während der zweiten Hälfte der Zugabe fiel ebenfalls entstehendes 6-Cyclopropyl-1,2-benzothiazol-3(2H)-on-1,1-dioxid (V) (28 g) aus, welches nach beendeter Zugabe der Base abfiltriert wurde. Das Filtrat wurde mit 11 n HCl versetzt und danach mit Dichlormethan extrahiert. Die organische Phase wurde getrocknet (Magnesiumsulfat) und ins Trockne eingedampft. Das Rohprodukt wurde zuerst mit Diisopropylether und dann mehrmals mit heißem Ethanol gewaschen. Der so entstandene Feststoff wurde anschließend im Vakuum getrocknet. Man erhielt so 44.4 g (50% d. Th.) 4-Cyclopropyl-2-{[(4-cyclopropyl-3-methoxy-5-oxo-4,5-dihydro-1H-1,2,4-triazol-1-yl)carbonyl]sulfamoyl}benzoesäuremethylester (Verbindung I-39).

Analog wurden beispielsweise in verschiedenen Ausbeuten die nachfolgenden Verbindungen hergestellt:
4-Cyclopropyl-2-{[(4-cyclopropyl-5-oxo-3-(2,2,2-trifluoroethoxy)-4,5-d ihydro-1H-1,2,4-triazol-1-yl)carbonyl]sulfamoyl}benzoesäureethylester (Verbindung I-115) (30% d. Th.)
4-Cyclopropyl-2-{[(3-methoxy-4-methyl-5-oxo-4,5-dihydro-1H-1,2,4-triazol-1 - yl)carbonyl]sulfamoyl}benzoesäurepropylester (Verbindung I-120) (43% d. Th.) 4-Cyclopropyl-2-{[(4-cyclopropyl-5-oxo-3-propoxy-4,5-dihydro-1H-1,2,4-triazol-1 - yl)carbonyl]sulfamoyl}benzoesäureisopropylester (Verbindung I-179) (55% d. Th.)

Die in der nachfolgenden Tabelle 1 beschriebenen Verbindungen der allgemeinen Formel (I) erhält man gemäß oder analog zu den oben beschriebenen Synthesebeispielen.

Bei Verbindungen, die einen oder mehrere Substituenten im Cyclopropanring tragen, kann es je nach Syntheseweg und eingesetzten Ausgangsverbindungen zur Bildung von Enantiomeren kommen. Diese sind ebenfalls Gegenstand der vorliegenden Erfindung.

**Tabelle 1: Verbindungen der Formel (I)**

| | | | | | |
|---|---|---|---|---|---|
| 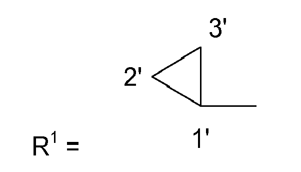 | | | | | |
| 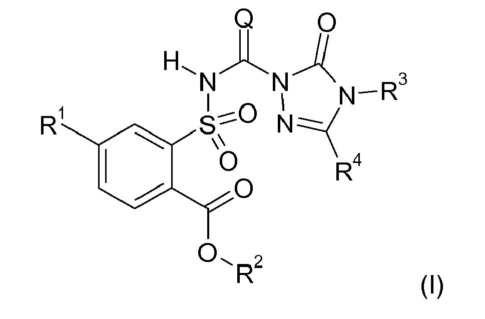 | | | | | |

| Bsp.-Nr. | R¹ | Q | R² | R³ | R⁴ |
|---|---|---|---|---|---|
| I-1 | Cyclopropyl | O | CH₃ | CH₃ | OCH₃ |
| I-2 | (1'-CH₃)-Cyclopropyl | O | CH₃ | CH₃ | OCH₃ |
| I-3 | (2'-CH₃)-Cyclopropyl | O | CH₃ | CH₃ | OCH₃ |
| I-4 | (2',2'-di-CH₃)-Cyclopropyl | O | CH₃ | CH₃ | OCH₃ |
| I-5 | (2',2'-di-F)-Cyclopropyl | O | CH₃ | CH₃ | OCH₃ |
| I-6 | (2',2'-di-Cl)-Cyclopropyl | O | CH₃ | CH₃ | OCH₃ |
| I-7 | (2'-F)-Cyclopropyl | O | CH₃ | CH₃ | OCH₃ |
| I-8 | (2'-CO₂CH₃)-Cyclopropyl | O | CH₃ | CH₃ | OCH₃ |
| I-9 | Cyclopropyl | O | CH₃ | CH₃ | OCH₂CH₃ |
| I-10 | (1'-CH₃)-Cyclopropyl | O | CH₃ | CH₃ | OCH₃ |
| I-11 | (2'-CH₃)-Cyclopropyl | O | CH₃ | CH₃ | OCH₃ |
| I-12 | (2',2'-di-CH₃)-Cyclopropyl | O | CH₃ | CH₃ | OCH₃ |
| I-13 | (2',2'-di-F)-Cyclopropyl | O | CH₃ | CH₃ | OCH₃ |
| I-14 | (2',2'-di-Cl)-Cyclopropyl | O | CH₃ | CH₃ | OCH₃ |
| I-15 | (2'-F)-Cyclopropyl | O | CH₃ | CH₃ | OCH₃ |
| I-16 | (2'-CO₂CH₃)-Cyclopropyl | O | CH₃ | CH₃ | OCH₃ |
| I-17 | Cyclopropyl | O | CH₃ | CH₃ | OCH₂CH₂CH₃ |
| I-18 | (2'-CH₃)-Cyclopropyl | O | CH₃ | CH₃ | OCH₂CH₂CH₃ |
| I-19 | (2',2'-di-CH₃)-Cyclopropyl | O | CH₃ | CH₃ | OCH₂CH₂CH₃ |
| I-20 | (2',2'-di-Cl)-Cyclopropyl | O | CH₃ | CH₃ | OCH₂CH₂CH₃ |
| I-21 | (2'-F)-Cyclopropyl | O | CH₃ | CH₃ | OCH₂CH₂CH₃ |
| I-22 | (2'-CO₂CH₃)-Cyclopropyl | O | CH₃ | CH₃ | OCH₂CH₂CH₃ |
| I-23 | Cyclopropyl | O | CH₃ | CH₃ | OCH(CH₃)₂ |
| I-24 | (2'-CH₃)-Cyclopropyl | O | CH₃ | CH₃ | OCH(CH₃)₂ |
| I-25 | (2',2'-di-CH₃)-Cyclopropyl | O | CH₃ | CH₃ | OCH(CH₃)₂ |
| I-26 | (2',2'-di-Cl)-Cyclopropyl | O | CH₃ | CH₃ | OCH(CH₃)₂ |
| I-27 | (2'-F)-Cyclopropyl | O | CH₃ | CH₃ | OCH(CH₃)₂ |
| I-28 | (2'-CO₂CH₃)-Cyclopropyl | O | CH₃ | CH₃ | OCH(CH₃)₂ |
| I-29 | Cyclopropyl | O | CH₃ | CH₃ | OCH₂CF₃ |
| I-30 | (2'-CH₃)-Cyclopropyl | O | CH₃ | CH₃ | OCH₂CF₃ |
| I-31 | (2',2'-di-CH₃)-Cyclopropyl | O | CH₃ | CH₃ | OCH₂CF₃ |
| I-32 | (2',2'-di-Cl)-Cyclopropyl | O | CH₃ | CH₃ | OCH₂CF₃ |
| I-33 | (2'-F)-Cyclopropyl | O | CH₃ | CH₃ | OCH₂CF₃ |
| I-34 | (2'-CO₂CH₃)-Cyclopropyl | O | CH₃ | CH₃ | OCH₂CF₃ |
| I-35 | Cyclopropyl | O | CH₃ | CH₃ | CH₃ |
| I-36 | Cyclopropyl | O | CH₃ | CH₃ | CH₂CH₃ |
| I-37 | Cyclopropyl | O | CH₃ | CH₃ | CH₂CH₂CH₃ |
| I-38 | Cyclopropyl | O | CH₃ | CH₃ | CH₂OCH₃ |
| I-39 | Cyclopropyl | O | CH₃ | Cyclopropyl | OCH₃ |
| I-40 | (1'-CH₃)-Cyclopropyl | O | CH₃ | Cyclopropyl | OCH₃ |
| I-41 | (2'-CH₃)-Cyclopropyl | O | CH₃ | Cyclopropyl | OCH₃ |
| I-42 | (2',2'-di-CH₃)-Cyclopropyl | O | CH₃ | Cyclopropyl | OCH₃ |
| I-43 | (2',2'-di-F)-Cyclopropyl | O | CH₃ | Cyclopropyl | OCH₃ |
| I-44 | (2',2'-di-Cl)-Cyclopropyl | O | CH₃ | Cyclopropyl | OCH₃ |
| I-45 | (2'-F)-Cyclopropyl | O | CH₃ | Cyclopropyl | OCH₃ |
| I-46 | (2'-CO₂CH₃)-Cyclopropyl | O | CH₃ | Cyclopropyl | OCH₃ |
| I-47 | Cyclopropyl | O | CH₃ | Cyclopropyl | OCH₂CH₃ |
| I-48 | (1'-CH₃)-Cyclopropyl | O | CH₃ | Cyclopropyl | OCH₂CH₃ |
| I-49 | (2'-CH₃)-Cyclopropyl | O | CH₃ | Cyclopropyl | OCH₂CH₃ |
| I-50 | (2',2'-di-CH₃)-Cyclopropyl | O | CH₃ | Cyclopropyl | OCH₂CH₃ |
| I-51 | (2',2'-di-F)-Cyclopropyl | O | CH₃ | Cyclopropyl | OCH₂CH₃ |
| I-52 | (2',2'-di-Cl)-Cyclopropyl | O | CH₃ | Cyclopropyl | OCH₂CH₃ |
| I-53 | (2'-F)-Cyclopropyl | O | CH₃ | Cyclopropyl | OCH₂CH₃ |
| I-54 | (2'-CO₂CH₃)-Cyclopropyl | O | CH₃ | Cyclopropyl | OCH₂CH₃ |
| I-55 | Cyclopropyl | O | CH₃ | Cyclopropyl | OCH₂CH₂CH₃ |
| I-56 | (2'-CH₃)-Cyclopropyl | O | CH₃ | Cyclopropyl | OCH₂CH₂CH₃ |
| I-57 | (2',2'-di-CH₃)-Cyclopropyl | O | CH₃ | Cyclopropyl | OCH₂CH₂CH₃ |
| I-58 | (2',2'-di-Cl)-Cyclopropyl | O | CH₃ | Cyclopropyl | OCH₂CH₂CH₃ |
| I-59 | (2'-F)-Cyclopropyl | O | CH₃ | Cyclopropyl | OCH₂CH₂CH₃ |
| I-60 | (2'-CO₂CH₃)-Cyclopropyl | O | CH₃ | Cyclopropyl | OCH₂CH₂CH₃ |
| I-61 | Cyclopropyl | O | CH₃ | Cyclopropyl | OCH(CH₃)₂ |
| I-62 | (2'-CH₃)-Cyclopropyl | O | CH₃ | Cyclopropyl | OCH(CH₃)₂ |
| I-63 | (2',2'-di-CH₃)-Cyclopropyl | O | CH₃ | Cyclopropyl | OCH(CH₃)₂ |
| I-64 | (2',2'-di-Cl)-Cyclopropyl | O | CH₃ | Cyclopropyl | OCH(CH₃)₂ |
| I-65 | (2'-F)-Cyclopropyl | O | CH₃ | Cyclopropyl | OCH(CH₃)₂ |
| I-66 | (2'-CO₂CH₃)-Cyclopropyl | O | CH₃ | Cyclopropyl | OCH(CH₃)₂ |
| I-67 | Cyclopropyl | O | CH₃ | Cyclopropyl | OCH₂CF₃ |
| I-68 | (2'-CH₃)-Cyclopropyl | O | CH₃ | Cyclopropyl | OCH₂CF₃ |
| I-69 | (2',2'-di-CH₃)-Cyclopropyl | O | CH₃ | Cyclopropyl | OCH₂CF₃ |
| I-70 | (2',2'-di-Cl)-Cyclopropyl | O | CH₃ | Cyclopropyl | OCH₂CF₃ |
| I-71 | (2'-F)-Cyclopropyl | O | CH₃ | Cyclopropyl | OCH₂CF₃ |
| I-72 | (2'-CO₂CH₃)-Cyclopropyl | O | CH₃ | Cyclopropyl | OCH₂CF₃ |
| I-73 | Cyclopropyl | O | CH₃ | Cyclopropyl | Cyclopropyl |
| I-74 | Cyclopropyl | O | CH₃ | Cyclopropyl | CH(CH₃)₂ |
| I-75 | Cyclopropyl | O | CH₃ | CH₂CH₃ | OCH₃ |
| I-76 | Cyclopropyl | O | CH₃ | CH₂CH₃ | OCH₂CH₃ |
| I-77 | Cyclopropyl | O | CH₂CH₃ | CH₃ | OCH₃ |
| I-78 | (2'-CH₃)-Cyclopropyl | O | CH₂CH₃ | CH₃ | OCH₃ |
| I-79 | (2',2'-di-CH₃)-Cyclopropyl | O | CH₂CH₃ | CH₃ | OCH₃ |
| I-80 | (2',2'-di-Cl)-Cyclopropyl | O | CH₂CH₃ | CH₃ | OCH₃ |
| I-81 | (2'-F)-Cyclopropyl | O | CH₂CH₃ | CH₃ | OCH₃ |
| I-82 | (2'-CO₂CH₃)-Cyclopropyl | O | CH₂CH₃ | CH₃ | OCH₃ |
| I-83 | Cyclopropyl | O | CH₂CH₃ | CH₃ | OCH₂CH₃ |
| I-84 | (2'-CH₃)-Cyclopropyl | O | CH₂CH₃ | CH₃ | OCH₂CH₃ |
| I-85 | (2',2'-di-CH₃)-Cyclopropyl | O | CH₂CH₃ | CH₃ | OCH₂CH₃ |
| I-86 | (2',2'-di-Cl)-Cyclopropyl | O | CH₂CH₃ | CH₃ | OCH₂CH₃ |
| I-87 | (2'-F)-Cyclopropyl | O | CH₂CH₃ | CH₃ | OCH₂CH₃ |
| I-88 | (2'-CO₂CH₃)-Cyclopropyl | O | CH₂CH₃ | CH₃ | OCH₂CH₃ |
| I-89 | Cyclopropyl | O | CH₂CH₃ | CH₃ | OCH₂CH₂CH₃ |
| I-90 | (2'-CH₃)-Cyclopropyl | O | CH₂CH₃ | CH₃ | OCH₂CH₂CH₃ |
| I-91 | (2',2'-di-CH₃)-Cyclopropyl | O | CH₂CH₃ | CH₃ | OCH₂CH₂CH₃ |
| I-92 | (2',2'-di-Cl)-Cyclopropyl | O | CH₂CH₃ | CH₃ | OCH₂CH₂CH₃ |
| I-93 | (2'-F)-Cyclopropyl | O | CH₂CH₃ | CH₃ | OCH₂CH₂CH₃ |
| I-94 | (2'-CO₂CH₃)-Cyclopropyl | O | CH₂CH₃ | CH₃ | OCH₂CH₂CH₃ |
| I-95 | Cyclopropyl | O | CH₂CH₃ | CH₃ | OCH(CH₃)₂ |
| I-96 | Cyclopropyl | O | CH₂CH₃ | CH₃ | OCH₂CF₃ |
| I-97 | Cyclopropyl | O | CH₂CH₃ | CH₃ | CH₃ |
| I-98 | Cyclopropyl | O | CH₂CH₃ | CH₃ | CH₂CH₃ |
| I-99 | Cyclopropyl | O | CH₂CH₃ | CH₃ | CH₂CH₂CH₃ |
| I-100 | Cyclopropyl | O | CH₂CH₃ | CH₃ | CH₂OCH₃ |
| I-101 | Cyclopropyl | O | CH₂CH₃ | Cyclopropyl | OCH₃ |
| I-102 | (2'-CH₃)-Cyclopropyl | O | CH₂CH₃ | Cyclopropyl | OCH₃ |
| I-103 | (2',2'-di-CH₃)-Cyclopropyl | O | CH₂CH₃ | Cyclopropyl | OCH₃ |
| I-104 | (2',2'-di-Cl)-Cyclopropyl | O | CH₂CH₃ | Cyclopropyl | OCH₃ |
| I-105 | (2'-F)-Cyclopropyl | O | CH₂CH₃ | Cyclopropyl | OCH₃ |
| I-106 | (2'-CO₂CH₃)-Cyclopropyl | O | CH₂CH₃ | Cyclopropyl | OCH₃ |
| I-107 | Cyclopropyl | O | CH₂CH₃ | Cyclopropyl | OCH₂CH₃ |
| I-108 | (2'-CH₃)-Cyclopropyl | O | CH₂CH₃ | Cyclopropyl | OCH₂CH₃ |
| I-109 | (2',2'-di-CH₃)-Cyclopropyl | O | CH₂CH₃ | Cyclopropyl | OCH₂CH₃ |
| I-110 | (2',2'-di-Cl)-Cyclopropyl | O | CH₂CH₃ | Cyclopropyl | OCH₂CH₃ |
| I-111 | (2'-F)-Cyclopropyl | O | CH₂CH₃ | Cyclopropyl | OCH₂CH₃ |
| I-112 | (2'-CO₂CH₃)-Cyclopropyl | O | CH₂CH₃ | Cyclopropyl | OCH₂CH₃ |
| I-113 | Cyclopropyl | O | CH₂CH₃ | Cyclopropyl | OCH₂CH₂CH₃ |
| I-114 | Cyclopropyl | O | CH₂CH₃ | Cyclopropyl | OCH(CH₃)₂ |
| I-115 | Cyclopropyl | O | CH₂CH₃ | Cyclopropyl | OCH₂CF₃ |
| I-116 | Cyclopropyl | O | CH₂CH₃ | Cyclopropyl | Cyclopropyl |
| I-117 | Cyclopropyl | O | CH₂CH₃ | Cyclopropyl | CH(CH₃)₂ |
| I-118 | Cyclopropyl | O | CH₂CH₃ | CH₂CH₃ | OCH₃ |
| I-119 | Cyclopropyl | O | CH₂CH₃ | OCH₂CH₃ | CH₂CH₃ |
| I-120 | Cyclopropyl | O | CH₂CH₂CH₃ | CH₃ | OCH₃ |
| I-121 | (2'-CH₃)-Cyclopropyl | O | CH₂CH₂CH₃ | CH₃ | OCH₃ |
| I-122 | (2',2'-di-CH₃)-Cyclopropyl | O | CH₂CH₂CH₃ | CH₃ | OCH₃ |
| I-123 | (2',2'-di-Cl)-Cyclopropyl | O | CH₂CH₂CH₃ | CH₃ | OCH₃ |
| I-124 | (2'-F)-Cyclopropyl | O | CH₂CH₂CH₃ | CH₃ | OCH₃ |
| I-125 | (2'-CO₂CH₃)-Cyclopropyl | O | CH₂CH₂CH₃ | CH₃ | OCH₃ |
| I-126 | Cyclopropyl | O | CH₂CH₂CH₃ | CH₃ | OCH₂CH₃ |
| I-127 | (2'-CH₃)-Cyclopropyl | O | CH₂CH₂CH₃ | CH₃ | OCH₂CH₃ |
| I-128 | (2',2'-di-CH₃)-Cyclopropyl | O | CH₂CH₂CH₃ | CH₃ | OCH₂CH₃ |
| I-129 | (2',2'-di-Cl)-Cyclopropyl | O | CH₂CH₂CH₃ | CH₃ | OCH₂CH₃ |
| I-130 | (2'-F)-Cyclopropyl | O | CH₂CH₂CH₃ | CH₃ | OCH₂CH₃ |
| I-131 | (2'-CO₂CH₃)-Cyclopropyl | O | CH₂CH₂CH₃ | CH₃ | OCH₂CH₃ |
| I-132 | Cyclopropyl | O | CH₂CH₂CH₃ | CH₃ | OCH₂CH₂CH₃ |
| I-133 | Cyclopropyl | O | CH₂CH₂CH₃ | CH₃ | OCH(CH₃)₂ |
| I-134 | Cyclopropyl | O | CH₂CH₂CH₃ | CH₃ | OCH₂CF₃ |
| I-135 | Cyclopropyl | O | CH₂CH₂CH₃ | CH₃ | CH₃ |
| I-136 | Cyclopropyl | O | CH₂CH₂CH₃ | CH₃ | CH₂CH₃ |
| I-137 | Cyclopropyl | O | CH₂CH₂CH₃ | CH₃ | CH₂CH₂CH₃ |
| I-138 | Cyclopropyl | O | CH₂CH₂CH₃ | CH₃ | CH₂OCH₃ |
| I-139 | Cyclopropyl | O | CH₂CH₂CH₃ | Cyclopropyl | OCH₃ |
| I-140 | (2'-CH₃)-Cyclopropyl | O | CH₂CH₂CH₃ | Cyclopropyl | OCH₃ |
| I-141 | (2',2'-di-CH₃)-Cyclopropyl | O | CH₂CH₂CH₃ | Cyclopropyl | OCH₃ |
| I-142 | (2',2'-di-Cl)-Cyclopropyl | O | CH₂CH₂CH₃ | Cyclopropyl | OCH₃ |
| I-143 | (2'-F)-Cyclopropyl | O | CH₂CH₂CH₃ | Cyclopropyl | OCH₃ |
| I-144 | (2'-CO₂CH₃)-Cyclopropyl | O | CH₂CH₂CH₃ | Cyclopropyl | OCH₃ |
| I-145 | Cyclopropyl | O | CH₂CH₂CH₃ | cyclopropyl | OCH₂CH₃ |
| I-146 | (2'-CH₃)-Cyclopropyl | O | CH₂CH₂CH₃ | Cyclopropyl | OCH₂CH₃ |
| I-147 | (2',2'-di-CH₃)-Cyclopropyl | O | CH₂CH₂CH₃ | Cyclopropyl | OCH₂CH₃ |
| I-148 | (2',2'-di-Cl)-Cyclopropyl | O | CH₂CH₂CH₃ | cyclopropyl | OCH₂CH₃ |
| I-149 | (2'-F)-Cyclopropyl | O | CH₂CH₂CH₃ | cyclopropyl | OCH₂CH₃ |
| I-150 | (2'-CO₂CH₃)-Cyclopropyl | O | CH₂CH₂CH₃ | Cyclopropyl | OCH₂CH₃ |
| I-151 | Cyclopropyl | O | CH₂CH₂CH₃ | Cyclopropyl | OCH₂CH₂CH₃ |
| I-152 | Cyclopropyl | O | CH₂CH₂CH₃ | Cyclopropyl | OCH(CH₃)₂ |
| I-153 | Cyclopropyl | O | CH₂CH₂CH₃ | Cyclopropyl | OCH₂CF₃ |
| I-154 | Cyclopropyl | O | CH₂CH₂CH₃ | Cyclopropyl | Cyclopropyl |
| I-155 | Cyclopropyl | O | CH₂CH₂CH₃ | Cyclopropyl | CH(CH₃)₂ |
| I-156 | Cyclopropyl | O | CH₂CH₂CH₃ | CH₂CH₃ | OCH₃ |
| I-157 | Cyclopropyl | O | CH₂CH₂CH₃ | OCH₂CH₃ | CH₂CH₃ |
| I-158 | Cyclopropyl | O | CH(CH₃)₂ | CH₃ | OCH₃ |
| I-159 | (2'-CH₃)-Cyclopropyl | O | CH(CH₃)₂ | CH₃ | OCH₃ |
| I-160 | (2',2'-di-CH₃)-Cyclopropyl | O | CH(CH₃)₂ | CH₃ | OCH₃ |
| I-161 | (2',2'-di-Cl)-Cyclopropyl | O | CH(CH₃)₂ | CH₃ | OCH₃ |
| I-162 | (2'-F)-Cyclopropyl | O | CH(CH₃)₂ | CH₃ | OCH₃ |
| I-163 | (2'-CO₂CH₃)-Cyclopropyl | O | CH(CH₃)₂ | CH₃ | OCH₃ |
| I-164 | Cyclopropyl | O | CH(CH₃)₂ | CH₃ | OCH₂CH₃ |
| I-165 | (2'-CH₃)-Cyclopropyl | O | CH(CH₃)₂ | CH₃ | OCH₂CH₃ |
| I-166 | (2',2'-di-CH₃)-Cyclopropyl | O | CH(CH₃)₂ | CH₃ | OCH₂CH₃ |
| I-167 | (2',2'-di-Cl)-Cyclopropyl | O | CH(CH₃)₂ | CH₃ | OCH₂CH₃ |
| I-168 | (2'-F)-Cyclopropyl | O | CH(CH₃)₂ | CH₃ | OCH₂CH₃ |
| I-169 | (2'-CO₂CH₃)-Cyclopropyl | O | CH(CH₃)₂ | CH₃ | OCH₂CH₃ |
| I-170 | Cyclopropyl | O | CH(CH₃)₂ | CH₃ | OCH₂CH₂CH₃ |
| I-171 | Cyclopropyl | O | CH(CH₃)₂ | CH₃ | OCH(CH₃)₂ |
| I-172 | Cyclopropyl | O | CH(CH₃)₂ | CH₃ | OCH₂CF₃ |
| I-173 | Cyclopropyl | O | CH(CH₃)₂ | CH₃ | CH₃ |
| I-174 | Cyclopropyl | O | CH(CH₃)₂ | CH₃ | CH₂CH₃ |
| I-175 | Cyclopropyl | O | CH(CH₃)₂ | CH₃ | CH₂CH₂CH₃ |
| I-176 | Cyclopropyl | O | CH(CH₃)₂ | CH₃ | CH₂OCH₃ |
| I-177 | Cyclopropyl | O | CH(CH₃)₂ | Cyclopropyl | OCH₃ |
| I-178 | Cyclopropyl | O | CH(CH₃)₂ | Cyclopropyl | OCH₂CH₃ |
| I-179 | Cyclopropyl | O | CH(CH₃)₂ | Cyclopropyl | OCH₂CH₂CH₃ |
| I-180 | Cyclopropyl | O | CH(CH₃)₂ | Cyclopropyl | OCH(CH₃)₂ |
| I-181 | Cyclopropyl | O | CH(CH₃)₂ | Cyclopropyl | OCH₂CF₃ |
| I-182 | Cyclopropyl | O | CH(CH₃)₂ | Cyclopropyl | Cyclopropyl |
| I-183 | Cyclopropyl | O | CH(CH₃)₂ | Cyclopropyl | CH(CH₃)₂ |
| I-184 | Cyclopropyl | O | CH(CH₃)₂ | CH₂CH₃ | OCH₃ |
| I-185 | Cyclopropyl | O | CH(CH₃)₂ | OCH₂CH₃ | CH₂CH₃ |
| I-186 | Cyclopropyl | O | CH₂CH₂CH₂CH₃ | CH₃ | OCH₃ |
| I-187 | Cyclopropyl | O | CH₂CH₂CH₂CH₃ | CH₃ | OCH₂CH₃ |
| I-188 | Cyclopropyl | O | CH₂CH₂CH₂CH₃ | Cyclopropyl | OCH₃ |
| I-189 | Cyclopropyl | O | CH₂CH₂CH₂CH₃ | cyclopropyl | OCH₂CH₃ |
| I-190 | Cyclopropyl | O | CH₂CH(CH₃)₂ | CH₃ | OCH₃ |
| I-191 | Cyclopropyl | O | CH₂CH(CH₃)₂ | CH₃ | OCH₂CH₃ |
| I-192 | Cyclopropyl | O | CH₂CH(CH₃)₂ | Cyclopropyl | OCH₃ |
| I-193 | Cyclopropyl | O | CH₂CH(CH₃)₂ | Cyclopropyl | OCH₂CH₃ |
| I-194 | Cyclopropyl | O | CH₂-cyclopropyl | CH₃ | OCH₃ |
| I-195 | Cyclopropyl | O | CH₂-cyclopropyl | CH₃ | OCH₂CH₃ |
| I-196 | Cyclopropyl | O | CH₂-cyclopropyl | Cyclopropyl | OCH₃ |
| I-197 | Cyclopropyl | O | CH₂-cyclopropyl | Cyclopropyl | OCH₂CH₃ |
| I-198 | Cyclopropyl | O | CH₂-CH=CH₂ | CH₃ | OCH₃ |
| I-199 | Cyclopropyl | O | CH₂-CH=CH₂ | CH₃ | OCH₂CH₃ |
| I-200 | Cyclopropyl | O | CH₂-CH=CH₂ | Cyclopropyl | OCH₃ |
| I-201 | Cyclopropyl | O | CH₂-CH=CH₂ | Cyclopropyl | OCH₂CH₃ |
| I-202 | Cyclopropyl | O | CH₂-C≡CH | CH₃ | OCH₃ |
| I-203 | Cyclopropyl | O | CH₂-C≡CH | CH₃ | OCH₂CH₃ |
| I-204 | Cyclopropyl | O | CH₂-C≡CH | Cyclopropyl | OCH₃ |
| I-205 | Cyclopropyl | O | CH₂-C≡CH | Cyclopropyl | OCH₂CH₃ |
| I-206 | Cyclopropyl | O | CH₂CH₂Cl | CH₃ | OCH₃ |
| I-207 | Cyclopropyl | O | CH₂CH₂Cl | CH₃ | OCH₂CH₃ |
| I-208 | Cyclopropyl | O | CH₂CH₂Cl | Cyclopropyl | OCH₃ |
| I-209 | Cyclopropyl | O | CH₂CH₂Cl | Cyclopropyl | OCH₂CH₃ |
| I-210 | Cyclopropyl | O | CH₂-Ph | CH₃ | OCH₃ |
| I-211 | Cyclopropyl | O | CH₂-Ph | CH₃ | OCH₂CH₃ |
| I-212 | Cyclopropyl | O | CH₂-Ph | Cyclopropyl | OCH₃ |
| I-213 | Cyclopropyl | O | CH₂-Ph | Cyclopropyl | OCH₂CH₃ |
| I-214 | Cyclopropyl | O | Ph | CH₃ | OCH₃ |
| I-215 | Cyclopropyl | O | Ph | CH₃ | OCH₂CH₃ |
| I-216 | Cyclopropyl | O | Ph | Cyclopropyl | OCH₃ |
| I-217 | Cyclopropyl | O | Ph | Cyclopropyl | OCH₂CH₃ |

¹H-NMR-Daten (300 oder 400 MHz, Solvens CDCl₃ oder [D₆]-DMSO, interner Standard: Tetrametylsilan δ = 0.00 ppm; br = breit(es); s = Singulett, d = Dublett, t = Triplett, dd = Doppeldublett, ddd = Dublett eines Doppeldubletts, m = Multiplett, q = Quartett, qnt = Quintett, sxt = Sextett, spt = Septett, t = Triplett, td = Triplett eines Dubletts
Verbindung I-1: ¹H-NMR (400MHz, CDCl₃): δ = 10.81 (br s, 1 H); 8.04 (d, J = 2.0, 1H); 7.62 (d, J = 8.1, 1H); 7.29 (dd, J = 2.0, 8.1, 1H); 4.08 (s, 3H); 3.97 (s, 3H); 3.18 (s, 3H); 2.00 (m, 1 H); 1.11 (m, 2H); 0.84 (m, 2H) ppm
Verbindung I-9: ¹H-NMR (400MHz, CDCl₃): δ = 10.82 (br s, 1 H); 8.04 (d, J = 2.0, 1 H); 7.62 (d, J = 7.8, 1 H); 7.29 (dd, J = 2.0, 8.1, 1 H); 4.46 (q, J = 7.1, 2H); 3.97 (s, 3H); 3.18 (s, 3H); 2.00 (m, 1H); 1.42 (t, J = 7.1, 3H); 1.10 (m, 2H); 0.84 (m, 2H) ppm
Verbindung I-17: ¹H-NMR (400MHz, CDCl₃): δ = 10.82 (br s, 1 H); 8.05 (d, J = 2.0, 1 H); 7.62 (d, J = 7.8, 1 H); 7.29 (dd, J = 2.0, 8.2, 1 H); 4.35 (t, J = 6.5, 2H); 3.97 (s, 3H); 3.18 (s, 3H); 2.00 (m, 1 H); 1.81 (m, 2H); 1.10 (m, 2H); 0.99 (t, J = 7.5, 3H); 0.84 (m, 2H) ppm
Verbindung I-23: ¹H-NMR (400MHz, CDCl₃): δ = 10.84 (br s, 1 H); 8.05 (d, J = 2.0, 1H); 7.62 (d, J = 7.8, 1H); 7.29 (dd, J = 2.0, 8.2, 1H); 5.18 (spt, J = 6.2, 1H); 3.97 (s, 3H); 3.15 (s, 3H); 2.00 (m, 1 H); 1.39 (d, J = 6.2, 6H); 1.10 (m, 2H); 0.84 (m, 2H) ppm
Verbindung I-29: ¹H-NMR (400MHz, CDCl₃): δ = 10.74 (br s, 1 H); 8.03 (d, J = 2.0, 1 H); 7.64 (d, J = 8.2, 1 H); 7.31 (dd, J = 1.6, 7.8, 1 H); 4.75 (q, J = 7.8, 2H); 3.97 (s, 3H); 3.25 (s, 3H); 2.01 (m, 1 H); 1.11 (m, 2H); 0.85 (m, 2H) ppm
Verbindung I-39: ¹H-NMR (400MHz, CDCl₃): δ = 10.81 (br s, 1 H); 8.04 (d, J = 2.0, 1 H); 7.62 (d, J = 8.1, 1 H); 7.29 (dd, J = 1.7, 7.8, 1 H); 4.07 (s, 3H); 3.97 (s, 3H); 2.71 (m, 1H); 2.00 (m, 1H); 1.10 (m, 2H); 1.01 (m, 4H); 0.84 (m, 2H) ppm
Verbindung I-47: ¹H-NMR (400MHz, CDCl₃): δ = 10.83 (br s, 1 H); 8.04 (d, J = 2.0, 1 H); 7.61 (d, J = 8.1, 1 H); 7.29 (dd, J = 2.0, 8.1, 1 H); 4.44 (q, J = 7.1, 2H); 3.97 (s, 3H); 2.71 (m, 1 H); 2.00 (m, 1H); 1.43 (t, J = 7.1, 3H); 1.10 (m, 2H); 1.01 (m, 4H); 0.84 (m, 2H) ppm
Verbindung I-55: ¹H-NMR (400MHz, CDCl₃): δ = 10.83 (br s, 1 H); 8.04 (d, J = 2.0, 1 H); 7.61 (d, J = 7.8, 1 H); 7.29 (dd, J = 2.0, 7.8, 1 H); 4.33 (t, J = 6.8, 2H); 3.97 (s, 3H); 2.71 (m, 1 H); 2.00 (m, 1H); 1.81 (m, 2H); 1.10 (m, 2H); 1.01 (m, 4H); 1.00 (t, J = 7.5, 3H); 0.84 (m, 2H) ppm
Verbindung I-61: ¹H-NMR (400MHz, CDCl₃): δ = 10.84 (br s, 1H); 8.05 (d, J = 1.6, 1H); 7.61 (d, J = 7.8, 1H); 7.28 (dd, J = 2.0, 8.2, 1H); 5.16 (spt, J = 6.2, 1H); 3.97 (s, 3H); 2.69 (m, 1 H); 2.00 (m, 1 H); 1.39 (d, J = 6.2, 6H); 1.10 (m, 2H); 0.99 (m, 4H); 0.84 (m, 2H) ppm
Verbindung I-67: ¹H-NMR (400MHz, CDCl₃): δ = 10.74 (br s, 1 H); 8.03 (d, J = 1.6, 1 H); 7.63 (d, J = 7.8, 1 H); 7.31 (dd, J = 2.0, 8.2, 1 H); 4.74 (q, J = 7.8, 2H); 3.97 (s, 3H); 2.77 ( m, 1H); 2.01 (m, 1 H); 1.10 (m, 4H); 1.02 (m, 2H); 0.84 (m, 2H) ppm
Verbindung 1-73: ¹H-NMR (400MHz, CDCl₃): δ = 11.01 (br s, 1 H); 7.83 (d, J = 1.6, 1H); 7.78 (d, J = 7.8, 1H); 7.28 (dd, J = 2.0, 8.2, 1H); 3.97 (s, 3H); 2.83 (m, 1H); 2.00 (m, 1H); 1.88 (m, 1H); 1.10 (m, 6H); 0.98 (m, 4H); 0.83 (m, 2H) ppm
Verbindung I-75: ¹H-NMR (400MHz, CDCl₃): δ = 10.81 (br s, 1 H); 8.05 (d, J = 2.0, 1H); 7.62 (d, J = 8.2, 1H); 7.29 (dd, J = 2.0, 8.2, 1H); 4.08 (s, 3H); 3.97 (s, 3H); 3.66 (q, J = 7.2, 2H); 2.00 (m, 1 H); 1.28 (t, J = 7.2, 3H); 1.10 (m, 2H); 0.84 (m, 2H) ppm
Verbindung I-76: ¹H-NMR (400MHz, CDCl₃): δ = 10.83 (br s, 1 H); 8.05 (d, J = 1.6, 1H); 7.62 (d, J = 7.8, 1H); 7.29 (dd, J = 2.0, 7.8, 1H); 4.46 (q, J = 7.2, 2H); 3.97 (s, 3H); 3.66 (q, J = 7.2, 2H); 2.00 (m, 1 H); 1.42 (t, J = 6.8, 3H); 1.28 (t, J = 7.2, 3H); 1.10 (m, 2H); 0.84 (m, 2H) ppm
Verbindung I-77: ¹H-NMR (400MHz, CDCl₃): δ = 10.76 (br s, 1H); 8.05 (d, J= 1.6, 1 H); 7.61 (d, J = 7.8, 1 H); 7.28 (dd, J = 1.6, 7.8, 1 H); 4.44 (q, J = 7.2, 2H); 4.07 (s, 3H); 3.18 (s, 3H); 2.00 (m, 1 H); 1.40 (t, J = 7.5, 3H); 1.10 (m, 2H); 0.84 (m, 2H) ppm
Verbindung I-83: ¹H-NMR (400MHz, CDCl₃): δ = 10.75 (br s, 1 H); 8.05 (d, J = 1.6, 1 H); 7.61 (d, J = 8.2, 1 H); 7.28 (dd, J = 1.6, 7.8, 1 H); 4.44 (q, J = 7.2, 2H); 3.17 (s, 3H); 2.00 (m, 1 H); 1.42 (t, J = 7.2, 3H); 1.40 (t, J = 7.2, 3H); 1.10 (m, 2H); 0.84 (m, 1 H) ppm
Verbindung I-101: ¹H-NMR (400MHz, CDCl₃): δ = 10.75 (br s, 1 H); 8.05 (d, J = 1.6, 1 H); 7.60 (d, J = 7.8, 1 H); 7.28 (dd, J = 2.0, 8.2, 1 H); 4.44 (q, J = 7.2, 2H); 4.06 (s, 3H); 2.70 (m, 1 H); 2.00 (m, 1 H); 1.40 (t, J = 7.2, 3H); 1.09 (m, 2H); 1.00 (m, 4H); 0.83 (m, 2H) ppm
Verbindung I-107: ¹H-NMR (400MHz, CDCl₃): δ = 10.73 (br s, 1 H); 8.05 (d, J = 2.0, 1 H); 7.60 (d, J = 7.8, 1 H); 7.29 (br t, J = 8.2, 1 H); 4.44 (m, 2H); 2.70 (m, 1 H); 1.99 (m, 1 H); 1.42 (t, J = 7.2, 3H); 1.40 (t, J = 7.2, 3H); 1.09 (m, 2H); 1.01 (m, 4H); 0.83 (m, 2H) ppm
Verbindung I-115: ¹H-NMR (400MHz, CDCl₃): δ = 10.70 (br s, 1 H); 8.03 (d, J = 2.0, 1H); 7.62 (d, J = 8.2, 1H); 7.30 (dd, J = 2.0, 8.2, 1H); 4.74 (q, J = 7.8, 2H); 4.44 (q, J = 7.2, 2H); 2.77 (m, 1 H); 2.00 (m, 1 H); 1.40 (t, J = 7.2, 3H); 1.09 (m, 4H); 1.01 (m, 1 H); 0.83 (m, 2H) ppm
Verbindung I-120: ¹H-NMR (400MHz, CDCl₃): δ = 10.76 (br s, 1 H); 8.05 (d, J = 1.3, 1H); 7.61 (d, J = 7.8, 1H); 7.28 (dd, J = 2.0, 8.5, 1H); 4.34 (t, J = 6.8, 2H); 4.07 (s, 3H); 3.17 (s, 3H); 2.00 (m, 1 H); 1.79 (sxt, J = 6.8, 2H); 1.10 (m, 2H); 1.00 (t, J = 7.2, 3H); 0.84 (m, 2H) ppm
Verbindung I-126: ¹H-NMR (400MHz, CDCl₃): δ = 10.77 (br s, 1 H); 8.05 (d, J = 1.6, 1 H); 7.61 (d, J = 7.8, 1 H); 7.28 (dd, J = 2.0, 8.2, 1 H); 4.45 (q, J = 7.2, 2H); 4.33 (t, J = 6.8, 2H); 3.18 (s, 3H); 2.00 (m, 1 H); 1.79 (sxt, J = 7.2, 2H); 1.42 (t, J = 7.2, 3H); 1.10 (m, 2H); 1.00 (t, J = 7.2, 3H); 0.84 (m, 2H) ppm
Verbindung I-132: ¹H-NMR (400MHz, CDCl₃): δ = 10.77 (br s, 1H); 8.05 (d, J = 1.6, 1H); 7.61 (d, J = 7.8, 1 H); 7.28 (dd, J = 2.0, 7.8, 1 H); 4.35 (t, J = 6.5, 2H); 4.34 (t, J = 6.8, 2H); 3.17 (s, 3H); 2.00 (m, 1H); 1.80 (m, 2H); 1.10 (m, 2H); 1.00 (t, J = 7.5, 3H); 0.99 (t, J = 7.5, 2H); 0.84 (m, 2H) ppm
Verbindung I-133: ¹H-NMR (400MHz, CDCl₃): δ = 10.78 (br s, 1 H); 8.06 (d, J = 1.6, 1H); 7.61 (d, J = 7.8, 1H); 7.28 (dd, J = 2.0, 7.8, 1H); 5.18 (spt, J = 6.2, 1H); 4.33 (t, J = 6.2, 2H); 3.14 (s, 3H); 2.00 (m, 1 H); 1.79 (sxt, J = 7.2, 2H); 1.39 (d, J = 6.2, 6H); 1.10 (m, 2H); 0.99 (t, J = 7.8, 3H); 0.84 (m, 2H) ppm
Verbindung I-139: ¹H-NMR (400MHz, CDCl₃): δ = 10.76 (br s, 1 H); 8.04 (d, J = 1.9, 1 H); 7.61 (d, J = 7.6, 1 H); 7.28 (dd, J = 1.9, 7.6, 1 H); 4.34 (t, J = 6.4, 2H); 4.06 (s, 3H); 2.70 (m, 1 H); 1.99 (m, 1 H); 1.79 (sxt, J = 7.0, 2H); 1.09 (m, 2H); 1.00 (m, 2H); 1.00 (t, J = 7.0, 3H); 0.83 (m, 2H) ppm
Verbindung I-145: ¹H-NMR (400MHz, CDCl₃): δ = 10.76 (br s, 1 H); 8.05 (d, J = 1.6, 1 H); 7.60 (d, J = 8.2, 1 H); 7.28 (dd, J = 1.6, 7.8, 1 H); 4.44 (q, J = 7.2, 2H); 4.33 (t, J = 6.8, 2H); 2.70 (m, 1 H); 1.99 (m, 1 H); 1.79 (sxt, J = 7.2, 2H); 1.42 (t, J = 6.8, 3H); 1.09 (m, 2H); 1.00 (m, 4H); 1.00 (t, J = 7.2, 3H); 0.83 (m, 2H) ppm
Verbindung I-151 : ¹H-NMR (400MHz, CDCl₃):δ = 10.76 (br s, 1H); 8.05 (d, J = 1.6, 1 H); 7.60 (d, J = 8.2, 1 H); 7.28 (dd, J = 2.0, 8.2, 1 H); 4.34 (t, J = 6.8, 2H); 4.33 (t, J = 6.5, 2H); 2.71 (m, 1 H); 2.00 (m, 2H); 1.80 (m, 4H); 1.09 (m, 2H); 1,00 (t, J = 7.2, 3H); 1.00 (m, 4H); 1.00 (t, J = 7.5, 3H); 0.83 (m, 2H) ppm
Verbindung I-152: ¹H-NMR (400MHz, CDCl₃): δ = 10.78 (br s, 1 H); 8.05 (d, J = 1.0, 1H); 7.61 (d, J = 8.2, 1H); 7.27 (m, 1H); 5.16 (m, 1H); 4.33 (t, J = 6.8, 1H); 2.68 (m, 1H); 1.99 (m, 1H); 1.79 (m, 2H); 1.39 (d, J = 6.2, 6H); 1.09 (m, 2H); 1.00 (m, 4H); 1.00 (t, J = 7.5, 3H); 0.83 (m, 2H) ppm
Verbindung I-153: ¹H-NMR (400MHz, CDCl₃): δ = 10.70 (br s, 1 H); 8.03 (d, J = 1.6, 1H); 7.62 (d, J = 7.8, 1H); 7.30 (dd, J = 2.0, 8.2, 1H); 4.73 (q, J = 7.8, 2H); 4.34 (t, J = 6.8, 2H); 2.76 (m, 1H); 2.00 (m, 1H); 1.80 (sxt, J = 7.5, 2H); 1.08 (m, 4H); 1.00 (m, 2H); 1.00 (t, J = 7.2, 2H); 0.84 (m, 2H) ppm
Verbindung I-158: ¹H-NMR (400MHz, CDCl₃): δ = 10.71 (br s, 1 H); 8.04 (d, J = 1.6, 1H); 7.56 (d, J = 8.2, 1 H); 7.27 (m, 1H); 5.33 (spt, J = 6.5, 1H); 4.07 (s, 3H); 3.17 (s, 3H); 1.99 (m, 1 H); 1.38 (d, J = 6.2, 6H); 1.09 (m, 2H); 0.83 (m, 2H) ppm
Verbindung I-164: ¹H-NMR (400MHz, CDCl₃): δ = 10.72 (br s, 1 H); 8.04 (d, J = 2.0, 1 H); 7.56 (d, J = 7.8, 1 H); 7.28 (dd, J = 1.6, 7.8, 1 H); 5.33 (spt, J = 6.2, 1 H); 4.45 (q, J = 6.8, 2H); 3.17 (s, 3H); 1.99 (m, 1 H); 1.42 (t, J = 7.2, 3H); 1.38 (d, J = 6.5, 6H); 1.09 (m, 2H); 0.83 (m, 2H) ppm
Verbindung I-170: ¹H-NMR (400MHz, CDCl₃): δ = 10.72 (br s, 1 H); 8.04 (d, J = 1.6, 1H); 7.55 (d, J = 8.2, 1H); 7.26 (m, 1H); 5.33 (m, 1H); 4.35 (t, J = 6.8, 2H); 3.17 (s, 3H); 1.99 (m, 1 H); 1.81 (m, 2H); 1.38 (d, J = 6.2, 6H); 1.09 (m, 2H); 0.99 (t, J = 7.5, 3H); 0.83 (m, 2H) ppm
Verbindung I-177: ¹H-NMR(400MHz, CDCl₃):δ = 10.70 (br s, 1H); 8.04 (d, J = 1.6, 1H); 7.55 (d, J = 7.8, 1 H); 7.27 (m, 1H); 5.33 (spt, J = 6.5, 1H); 4.06 (s, 3H); 2.70 (m, 1 H); 1.99 (m, 1 H); 1.38 (d, J= 6.2, 6H); 1.08 (m, 2H); 1.01 (m, 4H); 0.82 (m, 2H) ppm
Verbindung I-178: ¹H-NMR (400MHz, CDCl₃): δ = 10.71 (br s, 1H); 8.04 (d, J = 1.6, 1 H); 7.55 (d, J = 7.8, 1 H); 7.27 (dd, J = 1.6, 8.2, 1 H); 5.32 (spt, J = 6.2, 1 H); 4.44 (q, J = 6.8, 2H); 2.70 (m, 1 H); 1.99 (m, 1 H); 1.42 (t, J = 7.2, 3H); 1.38 (d, J = 6.5, 6H); 1.08 (m, 2H); 1.00 (m, 4H); 0.82 (m, 2H) ppm
Verbindung I-179: ¹H-NMR (400MHz, CDCl₃): δ = 10.71 (br s, 1 H); 8.04 (d, J = 1.6, 1H); 7.55 (d, J = 7.8, 1H); 7.27 (dd, J = 2.0, 7.8, 1H); 5.32 (spt, J = 6.2, 1H); 4.33 (t, J = 6.5, 2H); 2.71 (m, 1 H); 1.99 (m, 1 H); 1.81 (m, 2H); 1.38 (d, J = 6.2, 6H); 1.08 (m, 2H); 1.00 (m, 4H); 1.00 (t, J = 7.2, 3H); 0.82 (m, 2H) ppm
Verbindung I-180: ¹H-NMR (400MHz, CDCl₃): δ = 10.73 (br s, 1 H); 8.04 (d, J = 2.0, 1H); 7.55 (d, J = 8.2, 1H); 7.26 (dd, J = 2.0, 8.2, 1H); 5.32 (spt, J = 6.2, 1H); 5.15 (spt, J = 6.5, 1 H); 2.68 (m, 1 H); 1.99 (m, 1 H); 1.39 (d, J = 6.2, 6H); 1.38 (d, J = 5.9, 6H); 1.08 (m, 2H); 0.99 (m, 4H); 0.82 (m, 2H)
Verbindung I-181: ¹H-NMR (400MHz, CDCl₃): δ = 10.66 (br s, 1H); 8.02 (d, J = 1.6, 1 H); 7.57 (d, J = 7.8, 1 H); 7.29 (dd, J = 1.6, 7.8, 1 H); 5.33 (spt, J = 6.2, 1 H); 4.73 (q, J = 7.5, 2H); 2.76 (m, 1 H); 2.00 (m, 1 H); 1.38 (d, J = 6.2, 6H); 1.05 (m, 6H); 0.83 (m, 2H) ppm

### 3. Kalium-{[5-cyclopropyl-2-(methoxycarbonyl)phenyl]sulfonyl}[(4-cyclopropyl-3-methoxy-5-oxo-4,5-dihydro-1H-1,2,4-triazol-1-yl)carbonyl]azanid (Verbindung Ia-10)

0.2 g (0.46 mmol) 4-Cyclopropyl-2-{[(4-cyclopropyl-3-methoxy-5-oxo-4,5-dihydro-1H-1,2,4-triazol-1-yl)carbonyl]sulfamoyl}benzoesäuremethylester(Verbindung I-39) wurden in 2.5 ml Acetonitril vorgelegt. 0.03 g (0.48 mmol) Kaliumhydroxid gelöst in 2.5 ml Wasser wurden zugegeben. Die Reaktionslösung wurde über Nacht bei Raumtemperatur gerührt und anschließend ins Trockne eingedampft. Der Rückstand wurde bei 40°C im Hochvakuuum getrocknet. Man erhielt 0.2 g Kalium-{[5-cyclopropyl-2-(methoxycarbonyl)phenyl]sulfonyl}[(4-cyclopropyl-3-methoxy-5-oxo-4,5-dihydro-1H-1,2,4-triazol-1-yl)carbonyl]azanid (Verbindung Ia-10) (99% d. Th.).

Analog wurde beispielsweise die nachfolgende Verbindung hergestellt:
Natrium-{[5-cyclopropyl-2-(methoxycarbonyl)phenyl]sulfonyl}[(4-cyclopropyl-3-methoxy-5-oxo-4,5-dihydro-1H-1,2,4-triazol-1-yl)carbonyl]azanid (Verbindung Ia-9) (100% d.Th.)

Die in der nachfolgenden Tabelle 1a beschriebenen Verbindungen der allgemeinen Formel (Ia) erhält man gemäß oder analog zu den oben beschriebenen Synthesebeispielen.

Bei Verbindungen, die einen oder mehrere Substituenten im Cyclopropanring tragen, kann es je nach Syntheseweg und eingesetzten Ausgangsverbindungen zur Bildung von Enantiomeren kommen. Diese sind ebenfalls Gegenstand der vorliegenden Erfindung.

**Tabelle 1a: Verbindungen der Formel (Ia)**

| | | | | | | |
|---|---|---|---|---|---|---|
| 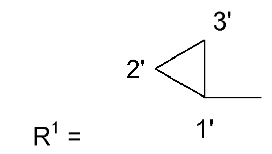 | | | | | | |
| 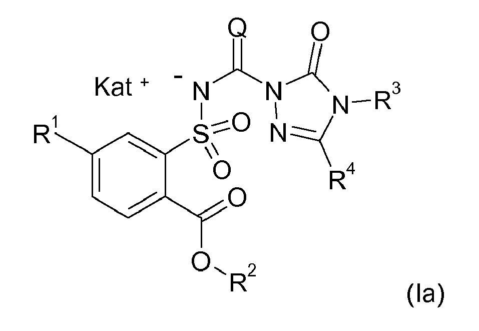 | | | | | | |

| Bsp.-Nr. | R¹ | Q | R² | Kat⁺ | R³ | R⁴ |
|---|---|---|---|---|---|---|
| Ia-1 | Cyclopropyl | O | CH₃ | Na⁺ | CH₃ | OCH₃ |
| Ia-2 | Cyclopropyl | O | CH₃ | K⁺ | CH₃ | OCH₃ |
| Ia-3 | Cyclopropyl | O | CH₃ | NH₄⁺ | CH₃ | OCH₃ |
| Ia-4 | Cyclopropyl | O | CH₃ | Bu₄N⁺ | CH₃ | OCH₃ |
| Ia-5 | Cyclopropyl | O | CH₃ | (HO(CH₂-CH₂))₃NH⁺ | CH₃ | OCH₃ |
| Ia-6 | Cyclopropyl | O | CH₃ | Na⁺ | CH₃ | OCH₂CH₃ |
| Ia-7 | Cyclopropyl | O | CH₃ | K⁺ | CH₃ | OCH₂CH₃ |
| Ia-8 | Cyclopropyl | O | CH₃ | NH₄⁺ | CH₃ | OCH₂CH₃ |
| Ia-9 | Cyclopropyl | O | CH₃ | Na⁺ | Cyclopropyl | OCH₃ |
| Ia-10 | Cyclopropyl | O | CH₃ | K⁺ | Cyclopropyl | OCH₃ |
| Ia-11 | Cyclopropyl | O | CH₃ | NH₄⁺ | Cyclopropyl | OCH₃ |
| Ia-12 | Cyclopropyl | O | CH₃ | Bu₄N⁺ | Cyclopropyl | OCH₃ |
| Ia-13 | Cyclopropyl | O | CH₃ | (HO(CH₂-CH₂))₃NH⁺ | Cyclopropyl | OCH₃ |
| Ia-14 | Cyclopropyl | O | CH₃ | Na⁺ | Cyclopropyl | OCH₂CH₃ |
| Ia-15 | Cyclopropyl | O | CH₃ | K⁺ | Cyclopropyl | OCH₂CH₃ |
| Ia-16 | Cyclopropyl | O | CH₃ | NH₄⁺ | Cyclopropyl | OCH₂CH₃ |
| Ia-17 | Cyclopropyl | O | CH₂CH₃ | Na⁺ | CH₃ | OCH₃ |
| Ia-18 | Cyclopropyl | O | CH₂CH₃ | K⁺ | CH₃ | OCH₃ |
| Ia-19 | Cyclopropyl | O | CH₂CH₃ | NH₄⁺ | CH₃ | OCH₃ |
| Ia-20 | Cyclopropyl | O | CH₂CH₃ | Bu₄N⁺ | CH₃ | OCH₃ |
| Ia-21 | Cyclopropyl | O | CH₂CH₃ | (HO(CH₂-CH₂))₃NH⁺ | CH₃ | OCH₃ |
| Ia-22 | Cyclopropyl | O | CH₂CH₃ | Na⁺ | CH₃ | OCH₂CH₃ |
| Ia-23 | Cyclopropyl | O | CH₂CH₃ | K⁺ | CH₃ | OCH₂CH₃ |
| Ia-24 | Cyclopropyl | O | CH₂CH₃ | NH₄⁺ | CH₃ | OCH₂CH₃ |
| Ia-25 | Cyclopropyl | O | CH₂CH₃ | Na⁺ | Cyclopropyl | OCH₃ |
| Ia-26 | Cyclopropyl | O | CH₂CH₃ | K⁺ | Cyclopropyl | OCH₃ |
| Ia-27 | Cyclopropyl | O | CH₂CH₃ | NH₄⁺ | Cyclopropyl | OCH₃ |
| Ia-28 | Cyclopropyl | O | CH₂CH₃ | Bu₄N⁺ | Cyclopropyl | OCH₃ |
| Ia-29 | Cyclopropyl | O | CH₂CH₃ | (HO(CH₂-CH₂))₃NH⁺ | Cyclopropyl | OCH₃ |
| Ia-30 | Cyclopropyl | O | CH₂CH₃ | Na⁺ | Cyclopropyl | OCH₂CH₃ |
| Ia-31 | Cyclopropyl | O | CH₂CH₃ | K⁺ | Cyclopropyl | OCH₂CH₃ |
| Ia-32 | Cyclopropyl | O | CH₂CH₃ | NH₄⁺ | Cyclopropyl | OCH₂CH₃ |

¹H-NMR-Daten (300 oder 400 MHz, Solvens CDCl₃ oder [D₆]-DMSO, interner Standard: Tetrametylsilan δ = 0.00 ppm; br = breit(es); s = Singulett, d = Dublett, t = Triplett, dd = Doppeldublett, ddd = Dublett eines Doppeldubletts, m = Multiplett, q = Quartett, qnt = Quintett, sxt = Sextett, spt = Septett, t = Triplett, td = Triplett eines Dubletts
Verbindung Ia-9: ¹H-NMR (400MHz, d₆-DMSO): δ = 7.74 (d, J = 1.6, 1H); 7.30 (d, J = 7.8, 1H); 7.17 (dd, J = 1.6, 7.8, 1H); 3.90 (s, 3H); 3.73 (s, 3H); 2.66 (m, 1H); 2.01 (m, 1H); 1.04 (m, 2H); 0.85 (m, 4H); 0.72 (m, 2H) ppm
Verbindung Ia-10: ¹H-NMR (400MHz, d₆-DMSO): δ = 7.74 (d, J = 2.0, 1H); 7.26 (d, J = 7.8, 1H); 7.15 (dd, J = 1.6, 7.8, 1H); 3.90 (s, 3H); 3.72 (s, 3H); 2.64 (m, 1H); 2.00 (m, 1H); 1.03 (m, 2H); 0.84 (m, 4H); 0.71 (m, 2H) ppm

### 4. 4-Cyclopropyl-2-sulfamoylbenzoesäuremethylester (Verbindung II-1)

### Methode 1

13 g (41.7 mmol) 2-tert-Butylsulfamoyl-4-cyclopropylbenzoesäuremethylester wurden in 45 ml Trifluoressigsäure gelöst und die Reaktionsmischung wurde 30 min bei Raumtemperatur gerührt. Danach wurde die Lösung mit Dichlormethan verdünnt, mit Wasser gewaschen, getrocknet (Magnesiumsulfat) und ins Trockne eingedampft. Man erhielt 10.4 g (98% d. Th.) 4-Cyclopropyl-2-sulfamoylbenzoesäuremethylester (Verbindung II-1).

### Methode 2

2.5 g (11.2 mmol) 6-Cyclopropyl-1,2-benzothiazol-3(2H)-on-1,1-dioxid (V) wurden in 25 ml 2-Methoxyethanol suspendiert. Unter Eiskühlung wurde 30 min HCl-Gas eingeleitet. Danach wurde die Reaktionsmischung bis auf Raumtemperatur erwärmt, 1 h bei Raumtemperatur und anschließend 4h unter Rückfluss gerührt. Die Mischung wurde ins Trockne eingedampft und der Rückstand dann in Dichlormethan aufgenommen. Die Lösung wurde zweimal mit Wasser gewaschen, die organische Phase getrocknet (Magnesiumsulfat) und ins Trockne einrotiert. Das Rohprodukt wurde mittels präp. HPLC gereinigt. Man erhielt 1.4 g (49% d. Th.) 4-Cyclopropyl-2-sulfamoylbenzoesäuremethylester (Verbindung II-1).

Analog wurden beispielsweise in verschiedenen Ausbeuten die nachfolgenden Verbindungen hergestellt:
4-Cyclopropyl-2-sulfamoylbenzoesäureethylester (Verbindung II-9) (24% d. Th.) durch Reaktion mit Ethanol
4-Cyclopropyl-2-sulfamoylbenzoesäurepropylester (Verbindung II-15) (44% d. Th.) durch Reaktion mit Propanol
4-Cyclopropyl-2-sulfamoylbenzoesäureisopropylester (Verbindung II-21) (34% d. Th.) durch Reaktion mit Isopropanol
4-Cyclopropyl-2-sulfamoylbenzoesäure-(2-chlorethyl)ester (Verbindung II-32) (25% d. Th.) durch Reaktion mit 2-Chlorethanol

**Tabelle 2: Verbindungen der Formel (II)**

| | | |
|---|---|---|
| 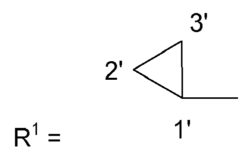 | | |
| 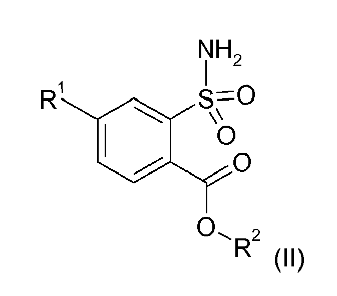 | | |

| Beisp.-Nr. | R¹ | R² |
|---|---|---|
| II-1 | Cyclopropyl | CH₃ |
| II-2 | (1'-CH3)-Cyclopropyl | CH₃ |
| II-3 | (2'-CH₃)-Cyclopropyl | CH₃ |
| II-4 | (2',2'-di-CH₃)-Cyclopropyl | CH₃ |
| II-5 | (2',2'-di-F)-Cyclopropyl | CH₃ |
| II-6 | (2',2'-di-Cl)-Cyclopropyl | CH₃ |
| II-7 | (2'-F)-Cyclopropyl | CH₃ |
| II-8 | (2'-CO₂CH₃)-Cyclopropyl | CH₃ |
| II-9 | Cyclopropyl | CH₂CH₃ |
| II-10 | (2'-CH₃)-Cyclopropyl | CH₂CH₃ |
| II-11 | (2',2'-di-CH₃)-Cyclopropyl | CH₂CH₃ |
| II-12 | (2',2'-di-Cl)-Cyclopropyl | CH₂CH₃ |
| II-13 | (2'-F)-Cyclopropyl | CH₂CH₃ |
| II-14 | (2'-CO₂CH₃)-Cyclopropyl | CH₂CH₃ |
| II-15 | Cyclopropyl | CH₂CH₂CH₃ |
| II-16 | (2'-CH₃)-Cyclopropyl | CH₂CH₂CH₃ |
| II-17 | (2',2'-di-CH₃)-Cyclopropyl | CH₂CH₂CH₃ |
| II-18 | (2',2'-di-Cl)-Cyclopropyl | CH₂CH₂CH₃ |
| II-19 | (2'-F)-Cyclopropyl | CH₂CH₂CH₃ |
| II-20 | (2'-CO₂CH₃)-Cyclopropyl | CH₂CH₂CH₃ |
| II-21 | Cyclopropyl | CH(CH₃)₂ |
| II-22 | (2'-CH₃)-Cyclopropyl | CH(CH₃)₂ |
| II-23 | (2',2'-di-CH₃)-Cyclopropyl | CH(CH₃)₂ |
| II-24 | (2',2'-di-Cl)-Cyclopropyl | CH(CH₃)₂ |
| II-25 | (2'-F)-Cyclopropyl | CH(CH₃)₂ |
| II-26 | (2'-CO₂CH₃)-Cyclopropyl | CH(CH₃)₂ |
| II-27 | Cyclopropyl | CH₂CH₂CH₂CH₃ |
| II-28 | Cyclopropyl | CH₂CH(CH₂)₃ |
| II-29 | Cyclopropyl | CH₂-cyclopropyl |
| II-30 | Cyclopropyl | CH₂-CH=CH₂ |
| II-31 | Cyclopropyl | CH₂-C≡CH |
| II-32 | Cyclopropyl | CH₂CH₂Cl |
| II-33 | Cyclopropyl | CH₂-Ph |
| II-34 | Cyclopropyl | Ph |

¹H-NMR-Daten (300 oder 400 MHz, Solvens CDCl₃ oder [D₆]-DMSO, interner Standard: Tetrametylsilan δ = 0.00 ppm; br = breit(es); s = Singulett, d = Dublett, t = Triplett, dd = Doppeldublett, ddd = Dublett eines Doppeldubletts, m = Multiplett, q = Quartett, qnt = Quintett, sxt = Sextett, spt = Septett, t = Triplett, td = Triplett eines Dubletts
Verbindung 11-1: ¹H-NMR (300MHz, CDCl₃): δ = 7.82 (d, J = 1.5, 1H); 7.79 (d, J = 8.1, 1H); 7.27 (dd, J = 1.8, 8.1, 1H); 5.80 (br s, 2H); 3.97 (s, 3H); 2.00 (m, 1H); 1.13 (m, 2H); 0.83 (m, 2H) ppm
Verbindung 11-2: ¹H-NMR (400MHz, CDCl₃): δ = 7.81 (d, J = 1.9, 1H); 7.77 (d, J = 8.3, 1H); 7.27 (dd, J = 1.9, 7.6, 1H); 5.78 (br s, 2H); 4.42 (q, J = 7.0, 2H); 2.00 (m, 1H); 1.41 (t, J = 7.0, 3H); 1.12 (m, 2H); 0.82 (m, 2H) ppm
Verbindung 11-3: ¹H-NMR (400MHz, CDCl₃): δ = 7.82 (d, J = 1.9, 1H); 7.77 (d, J = 7.6, 1H); 7.28 (dd, J = 1.9, 7.6, 1H); 5.78 (br s, 2H); 4.33 (t, J = 7.0, 2H); 2.00 (m, 1H); 1.81 (m, 2H); 1.12 (m, 2H); 1.03 (t, J = 7.0, 3H); 0.82 (m, 2H) ppm
Verbindung 11-4: ¹H-NMR (400MHz, CDCl₃): δ = 7.80 (d, J = 1.3, 1H); 7.73 (d, J = 7.6, 1H); 7.27 (dd, J = 1.9, 7.6, 1H); 5.78 (br s, 2H); 5.28 (spt, J = 6.4, 1H); 2.00 (m, 1H); 1.39 (d, J = 6.4, 6H); 1.11 (m, 2H); 0.81 (m, 2H) ppm
Verbindung 11-10: ¹H-NMR (400MHz, CDCl₃): δ = 7.84 (m, 2H); 7.30 (dd, J = 1.9, 7.6, 1H); 5.70 (br s, 2H); 4.64 (m, 2H); 3.84 (m, 2H); 2.01 (m, 1H); 1.14 (m, 2H); 0.84 (m, 2H) ppm

### 5. 2-tert-Butylsulfamoyl-4-cyclopropylbenzoesäuremethylester

1.04 g (45 mmol) Natrium wurden in 84 ml wasserfreiem Methanol gelöst. Dann wurden bei Raumtemperatur 6.3 (22.6 mmol) 2-tert-Butyl-6-cyclopropyl-1,2-benzothiazol-3(2H)-on-1,1-dioxid zugegeben. Nach 25 min Rühren bei Raumtemperatur wurde die Reaktionsmischung auf HCl-saures Eiswasser (ca. 50 ml H₂O/konz. HCl 8:2) gegossen. Nach Extraktion mit Dichlormethan wurde die organische Phase mit Wasser gewaschen, getrocknet (Magnesiumsulfat) und ins Trockne eingedampft. Man erhielt 6.3 g (88% d. Th.) 2-tert-Butylsulfamoyl-4-cyclopropylbenzoesäuremethylester.

¹H-NMR (300MHz, CDCl₃): δ = 7.81 (d, J = 1.5, 1H); 7.74 (d, J = 7.9, 1H); 7.22 (dd, J = 1.5, 7.9, 1H); 6.36 (br s, 1H); 3.95 (s, 3H); 2.00 (m, 1H); 1.28 (s, 9H); 1.12 (m, 2H); 0.82 (m, 2H) ppm.

### 6. 2-tert-Butyl-6-cyclopropyl-1,2-benzothiazol-3(2H)-on-1,1 -dioxid

### Methode 1

20 g (50 mmol) 2-tert-Butylsulfamoyl-4-iodbenzoesäuremethylester wurden in 120 ml Toluol gelöst und mit 17.3 g (201 mmol) Cyclopropylboronsäure, 32.1 g (151 mmol) Kaliumphosphat, 1.1 g (5 mmol) Palladiumacetat und 1.4 g (5 mmol) Tricyclohexylphosphin versetzt. Die Reaktionsmischung wurde 100h unter Rückfluss gerührt. Die während der Reaktion entstandenen Feststoffe wurden abfiltriert, die Reaktionsmischung wurde in Ethylacetat aufgenommen und mehrfach mit Wasser gewaschen. Nach Trocknung (Magnesiumsulfat) wurde die organische Phase ins Trockne eingedampft. Man erhielt 13.8 g (98% d. Th.) 2-tert-Butyl-6-cyclopropyl-1,2-benzothiazol-3(2H)-on-1,1-dioxid.
¹H-NMR (400MHz, CDCl₃): δ = 7.82 (m, 1H); 7.43 (m, 2H); 2.06 (m, 1H); 1.76 (s, 9H); 1.18 (m, 2H); 0.86 (m, 2H) ppm.

### Methode 2

1 g (3.2 mmol) 2-tert-Butylsulfamoyl-4-cyclopropylbenzoesäuremethylester wurde in 20 ml Ethanol gelöst und unter Rühren mit 0.63 g (11.2 mmol) gepulvertem Kaliumhydroxid versetzt. Die Reaktionsmischung wurde 4h unter Rückfluss gerührt. Danach wurde das Lösungsmittel destillativ entfernt und der Rückstand in Wasser aufgenommen. Mittels Zugabe von 2N HCl wurde ein pH-Wert von 2 erreicht. Danach wurde mit Diethylether gewaschen, die organische Phase mit Magnesiumsulfat getrocknet und ins Trockne eingedampft. Man erhielt 0.95 g (95% d. Th.) 2-(tert-Butylsulfamoyl)-4-cyclopropylbenzoesäure.
¹H-NMR (400MHz, CDCl₃): δ = 7.88 (d, J = 8.2, 1H); 7.85 (d, J = 1.6, 1H); 7.26 (dd, J = 1.6, 7.8, 1H); 6.25 (br s, 1H); 5.5 - 4.5 (br s, 1H); 2.01 (m, 1H); 1.26 (s, 9H); 1.14 (m, 2H); 0.83 (m, 2H).
0.95 g (3.2 mmol) 2-(tert-Butylsulfamoyl)-4-cyclopropylbenzoesäure wurden in 4.6 ml Thionylchlorid 3h bei 70°C gerührt. Danach wurde ins Trockne eingedampft und der Rückstand wurde zweimal in Toluol verrührt und eingedampft. Danach wurde der Rückstand in Dichlormethan gelöst und mit wenig Wasser gewaschen. Die organische Phase wurde getrocknet (Magnesiumsulfat) und ins Trockne eingedampft. Man erhielt 0.87 g (96% d. Th.) 2-tert-Butyl-6-cyclopropyl-1,2-benzothiazol-3(2H)-on-1,1-dioxid.

### 7. 6-Cyclopropyl-1,2-benzothiazol-3(2H)-on-1,1-dioxid (V)

0.87 g (3.1 mmol) 2-tert-Butyl-6-cyclopropyl-1,2-benzothiazol-3(2H)-on-1,1-dioxid wurden in 7.2 ml Trifluoressigsäure gelöst und 24h bei 70°C gerührt. Danach wurde die Reaktionsmischung ins Trockne eingedampft. Der Rückstand wurde mit Diisopropylether verrührt und die Suspension wurde filtriert. Der Filterrückstand wurde mit Ether nachgewaschen und getrocknet. Man erhielt 0.44 g (63% d. Th.) 6-Cyclopropyl-1,2-benzothiazol-3(2H)-on-1,1-dioxid (V), welches 94%ige Reinheit aufwies. Durch Eindampfen der Filtrate erhielt man weitere 0.26 g Produkt in 86%iger Reinheit.
¹H-NMR (400MHz, CDCl₃): δ = 7.89 (d, J = 7.8, 1H); 7.51 (br s, 1H); 7.50 (dd, J = 1.6, 8.2, 1H); 2.10 (m, 1H); 1.20(m, 2H); 0.89(m, 2H)ppm.

Die erfindungsgemäßen Verbindungen der allgemeinen Formel (I) und/oder deren Salze gemäß der allgemeinen Formel (Ia) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.
Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.
Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.
Obgleich die erfindungsgemäßen Verbindungen der allgemeinen Formel (I) und/oder deren Salze gemäß der allgemeinen Formel (Ia) eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.
Darüber hinaus weisen die erfindungsgemäßen Verbindungen der allgemeinen Formel (I) und/oder deren Salze gemäß der allgemeinen Formel (Ia) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Aufgrund ihrer herbiziden und pflanzenwachstumsregulatorischen Eigenschaften können die erfindungsegemäßen Verbindungen der allgemeinen Formel (I) und/oder deren Salze gemäß der allgemeinen Formel (Ia) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmter Herbizide, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Aufgrund ihrer herbiziden und pflanzenwachstumsregulatorischen Eigenschaften können die erfindungsgemäßen Verbindungen der allgemeinen Formel (I) und/oder deren Salze gemäß der allgemeinen Formel (Ia) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) und/oder deren Salze gemäß der allgemeinen Formel (Ia) in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der allgemeinen Formel (I) und/oder deren Salze gemäß der allgemeinen Formel (Ia) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant),.
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) und/oder deren Salze gemäß der allgemeinen Formel (Ia) in transgenen Kulturen eingesetzt werden, welche gegen Wuchsstoffe, wie z. B. 2,4-D, Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum^{™} GAT^{™} (Glyphosate ALS Tolerant) eingesetzt werden.

Bei der Anwendung der erfindungsgemäßen Wirkstoffe der allgemeinen Formel (I) und/oder deren Salze gemäß der allgemeinen Formel (Ia) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinothricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinothricin, z.B. Raps), IMI® (Toleranz gegen Imidazolinone) und STS® (Toleranz gegen Sulfonylharnstoffe z.B. Mais) vertrieben werden. Als Herbizidresistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").
Die aufgeführten Pflanzen können besonders vorteilhaft erfindungsgemäß mit den Verbindungen der allgemeinen Formel (I) und/oder deren Salze gemäß der allgemeinen Formel (Ia) bzw. den erfindungsgemäßen Wirkstoffmischungen behandelt werden, wobei zusätzlich zu der guten Bekämpfung der Unkrautpflanzen die oben genannten synergistischen Effekte mit den transgenen Pflanzen oder Pflanzensorten auftreten. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Gegenstand der Erfindung ist deshalb auch die Verwendung der erfindungsgemäßen Verbindungen der allgemeinen Formel (I) und/oder deren Salze gemäß der allgemeinen Formel (Ia) als Herbizide zur Bekämpfung von Schadpflanzen in transgenen Kulturpflanzen.

Die erfindungsgemäßen Verbindungen der allgemeinen Formel (I) und/oder deren Salze gemäß der allgemeinen Formel (Ia) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen der allgemeinen Formel (I) und/oder deren Salze gemäß der allgemeinen Formel (Ia) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖL-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

### B. Formulierungsbeispiele

a) Ein Stäubemittel wird erhalten, indem man 10 Gew.-Teile einer Verbindung der allgemeinenformel (I) und/oder deren Salze gemäß der allgemeinen Formel (Ia) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der allgemeinen Formel (I) und/oder deren Salze gemäß der allgemeinen Formel (Ia), 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der allgemeinen Formel (I) und/oder deren Salze gemäß der allgemeinen Formel (Ia) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der allgemeinen Formel (I) und/oder deren Salze gemäß der allgemeinen Formel (Ia), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man 75 Gew.-Teile einer Verbindung der allgemeinen Formel (I) und/oder deren Salze gemäß der allgemeinen Formel (Ia),
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gew.-Teile einer Verbindung der allgemeinen Formel (I) und/oder deren Salze gemäß der allgemeinen Formel (Ia),
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

Die erfindungsgemäßen Verbindungen der allgemeinen Formel (I) und/oder deren Salze gemäß der allgemeinen Formel (Ia) können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Als Kombinationspartner für die erfindungsgemäßen Verbindungen der allgemeinen Formel (I) und/oder deren Salze gemäß der allgemeinen Formel (Ia) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, Bencarbazone, Benfluralin, Benfuresate, Bensulide, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifopbutyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indaziflam, Indolessigsäure (IAA), 4-Indol-3-ylbuttersäure (IBA), Iodosulfuron, Iodosulfuron-methyl-natrium, Ioxynil, Ipfencarbazone,Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazosulfuron, Methazole, Methiozolin, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapacethyl, Tritosulfuron, Tsitodef, Uniconazole, Uniconazole-P, Vernolate, ZJ-0166, ZJ-0270, ZJ-0543, ZJ-0862 sowie die folgenden Verbindungen

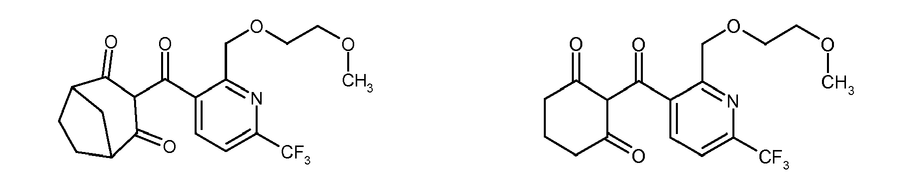

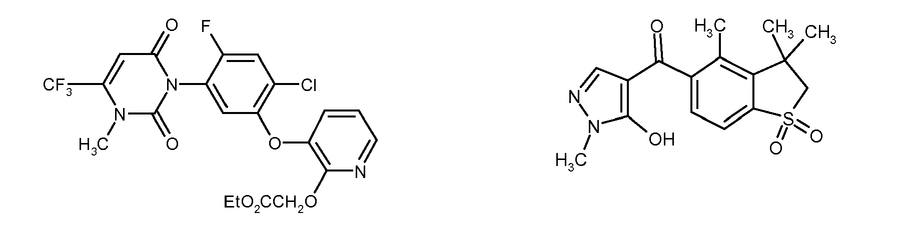

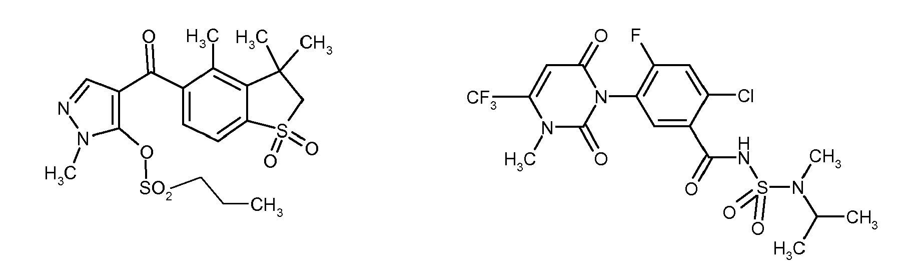

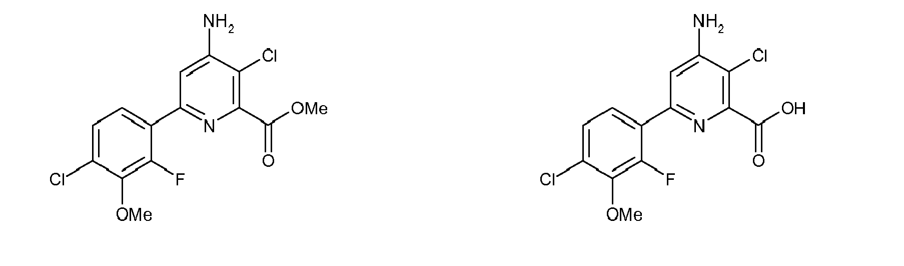

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) und/oder deren Salze gemäß der allgemeinen Formel (Ia) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) und/oder deren Salze gemäß der allgemeinen Formel (Ia) von besonderem Interesse, welche die Verbindungen der allgemeinen Formel (I) und/oder deren Salze gemäß der allgemeinen Formel (Ia) bzw. deren Kombinationen mit anderen Herbiziden oder Pestiziden und Safenern enthalten.

Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen der allgemeinen Formel (I) und/oder deren Salze gemäß der allgemeinen Formel (Ia) alleinig oder aber in deren Kombinationen mit weiteren Pestiziden in Frage:
S1) Verbindungen der Formel (S1), 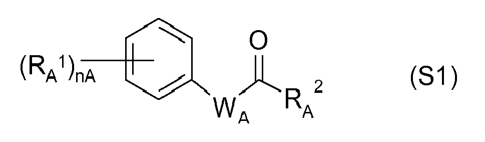 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl,
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 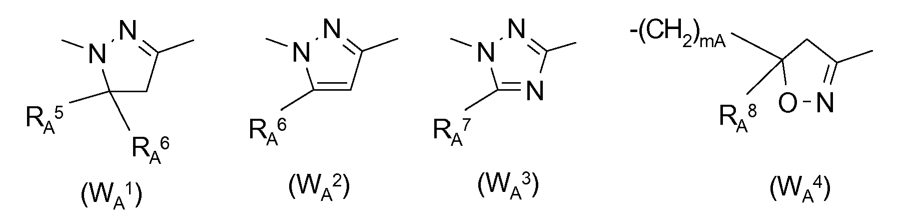
   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 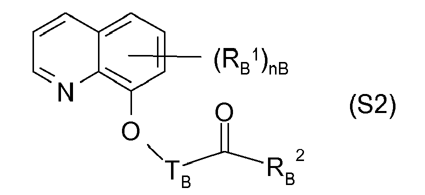 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl,
   - n_{B}: ist eine natürliche Zahl von 0 bis 5, vorzugsweise 0 bis 3;
   - R_{B}²: ist OR_{B}³, SR_{B}³ oder NR_{B}³ R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 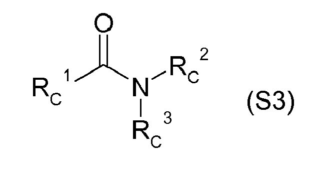 wobei die Symbole und Indizes folgende Bedeutungen haben:
   - R_{c}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{c}², R_{c}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{c}² und R_{c}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF,
   "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 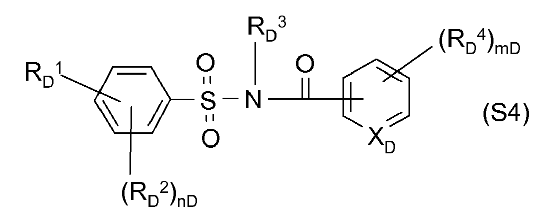 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend V_{D} Heteroatome aus der Gruppe Stickstoff, Sauerstoff und Schwefel, wobei die sieben letztgenannten Reste durch V_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch V_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem dem sie tragenden Stickstoffatom einen Pyrrolidinyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch V_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - v_{D}: ist 0,1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 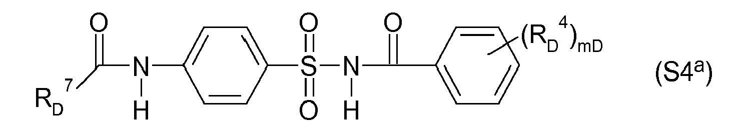 worin
   - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - R_{D}⁴: Halogen, (C₁-C₄)Alkyl, (C₁-C₄)Alkoxy, CF₃;
   - m_{D}: 1 oder 2;
   - v_{D}: ist 0,1,2 oder 3 bedeutet;
   sowie
   Acylsulfamoylbenzoesäureamide, z.B. der nachfolgenden Formel (S4^{b}), die z.B. bekannt sind aus WO-A-99/16744, 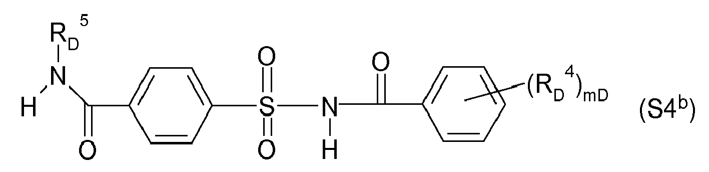 z.B. solche worin
   R_{D}⁵ = Cyclopropyl und (R_{D}⁴) = 2-OMe ist ("Cyprosulfamide", S4-1),
   R_{D}⁵ = Cyclopropyl und (RD⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 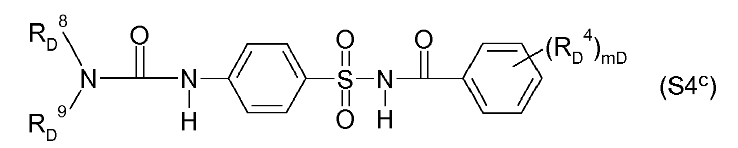 worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF₃
   m_{D} 1 oder 2 bedeutet; beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff.
S5) Wirkstoffe aus der Klasse der Hydroxyaromaten und der aromatischaliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 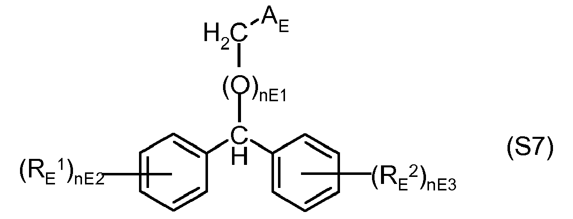 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8),wie sie in der WO-A-98/27049 beschrieben sind 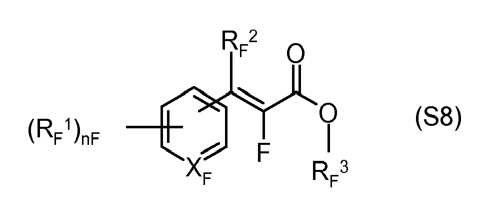 worin
   - X_{F}: CH oder N,
   - n_{F}: für den Fall, dass X_{F}=N ist, eine ganze Zahl von 0 bis 4 und für den Fall, dass X_{F}=CH ist, eine ganze Zahl von 0 bis 5 ,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   - X_{F}: CH,
   - n_{F}: eine ganze Zahl von 0 bis 2 ,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolylcarbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}) wie sie in der WO-A-2007/023719 und WO-A-2007/023764 beschrieben sind 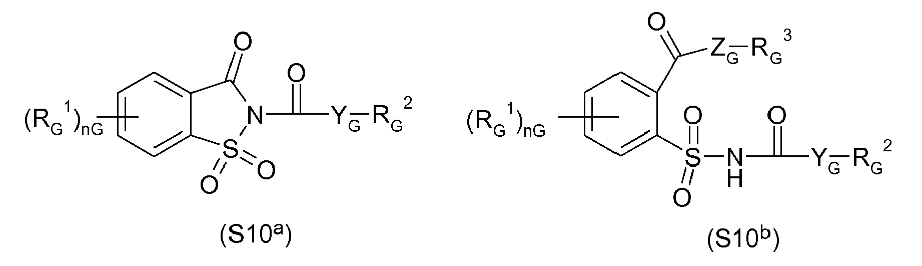 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1 H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG-838" (CAS-Reg.Nr. 133993-74-5)
   (2-propenyl 1-oxa-4-azaspiro[4.5]decan-4-carbodithioat) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (S-1-Methyl-1-phenylethyl-piperidin-1-carbothioat), das als Safener für Reis gegen Schäden des Herbizids Molinate bekannt ist, "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist, "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere wie sie in der WO-A-2008/131861 und WO-A-2008/131860 beschrieben sind 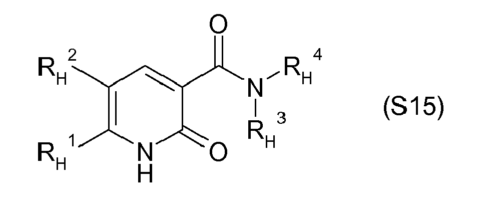 worin
   - R_{H}¹: einen (C₁-C₆)Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)Alkyl, (C₂-C₁₆)Alkenyl
   oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet und
   - R_{H}⁴: Wasserstoff oder (C₁-C₄)-Alkyl bedeutet oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Besonders bevorzugte Kombinationen von herbiziden Wirkstoffen (A) wie in Tabelle 1 oder der Tabelle 1 a benannt und Safenern (B) sind solche, bei denen der Safener (B) ausgewählt ist aus der Gruppe von Safenern bestehend aus den Verbindungen der Formeln S1-1 (Mefenpyr-diethyl), S1-11 (Isoxadifen-ethyl), S2-1 (Chloquintocetmexyl), S13-2 (Fenclorim), S4-1 (4-Cyclopropylaminocarbonyl-N-(2-methoxybenzoyl)benzolsulfonamid, N-({4-[(cyclopropylamino)carbonyl]phenyl}sulfonyl)-2-methoxybenzamide; Cyprosulfamide), ganz besonders bevorzugt als Safener (B) sind Verbindungen S1-1 und S4-1).

Für die Anwendung in Reis besonders bevorzugt ist Isoxadifen-ethyl. Für die Anwendung in Getreide besonders bevorzugt sind Mefenpyr-diethyl, Cloquintocet-mexyl und Cyprosulfamide, in Mais insbesondere Isoxadifen-ethyl und Cyprosulfamide. Für die Anwendung in Zuckerrohr bevorzugt ist Isoxadifen-ethyl.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Einige der Safener sind bereits als Herbizide bekannt und entfalten somit neben der Herbizidwirkung bei Schadpflanzen zugleich auch Schutzwirkung bei den Kulturpflanzen.
Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der allgemeinen Formel (I) und/oder deren Salze gemäß der allgemeinen Formel (Ia) oder deren Mischungen mit weiteren Herbiziden/Pestiziden formuliert werden und als Fertigformulierung oder Tankmischung mit den Herbiziden bereitgestellt und angewendet werden.

Mit den äußeren Bedingungen wie Temperatur, Feuchtigkeit, der Art des verwendeten Herbizids, u.a. variiert die erforderliche Aufwandmenge der Verbindungen der allgemeinen Formel (I) und/oder deren Salze gemäß der allgemeinen Formel (Ia). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der allgemeinen Formel (I) und/oder deren Salze gemäß der allgemeinen Formel (Ia) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen, besseres Wachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

### C. Biologische Beispiele

### 1. Unkrautwirkung im Vorauflauf

Samen bzw. Rhizomstücke von mono- und dikotylen Unkrautpflanzen wurden in Papptöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wässrige Suspensionen bzw. Emulsionen mit einer Wasseraufwandmenge von umgerechnet 600 bis 800 I/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Ergebnisse zeigen, weisen erfindungsgemäße Verbindungen eine gute herbizide Vorauflaufwirksamkeit gegen ein breites Spektrum von Ungräsern und Unkräutern auf.

Nachfolgend sind beispielhaft die Wirksamkeiten einiger beanspruchter Verbindungen auf verschiedene getestete Unkräuter in % dargestellt.

Es bedeuten:

| | | |
|---|---|---|
| 0% | = | keine Wirkung (wie unbehandelte Konrolle) |
| 100% | = | totale Vernichtung |

Es bedeuten:

| | | |
|---|---|---|
| ALOMY | = | Alopecurus agrestis |
| ABUTH | = | Abutilon theophrasti |
| ECHCG | = | Echinochloa crus-galli |
| LOLMU | = | Lolium multiflorum |
| SETVI | = | Seteria viridis |
| MATIN | = | Matricaria indora |
| POLCO | = | Polygonum convolvulus |
| STEME | = | Stellaria media |
| VERPE | = | Veronica persica |

| Verbindung | Applikation | A | E | L | A | P | M |
|---|---|---|---|---|---|---|---|
| | | L | C | O | B | O | A |
| | ga.i./ha | O | H | L | U | L | T |
| | | M | C | M | T | C | I |
| | | Y | G | U | H | O | N |
| I-1 | 80 | 90 | 90 | 90 | 90 | 90 | 90 |
| I-1 | 20 | 90 | 90 | 90 | 90 | 90 | 90 |

| Verbindung | Applikation | A | E | L | S | A | P | M | V |
|---|---|---|---|---|---|---|---|---|---|
| | | L | C | O | E | B | O | A | E |
| | ga.i./ha | O | H | L | T | U | L | T | R |
| | | M | C | M | V | T | C | I | P |
| | | Y | G | U | I | H | O | N | E |
| I-9 | 80 | 90 | 90 | 90 | 90 | 90 | 90 | 90 | 90 |
| I-9 | 20 | 90 | 90 | 90 | 90 | 90 | 90 | 90 | 90 |

| Verbindung | Applikation | A | A | A | M | P | S | V |
|---|---|---|---|---|---|---|---|---|
| | | L | B | M | A | H | T | E |
| | ga.i./ha | O | U | A | T | B | E | R |
| | | M | T | R | I | P | M | P |
| | | Y | H | E | N | U | E | E |
| I-17 | 80 | 100 | 100 | 100 | 100 | 100 | 100 | 100 |
| I-17 | 20 | 100 | 90 | 100 | 90 | 90 | 100 | 90 |

| Verbindung | Applikation | A | A | A | M | P | S | V |
|---|---|---|---|---|---|---|---|---|
| | | L | B | M | A | H | T | E |
| | ga.i./ha | O | U | A | T | B | E | R |
| | | M | T | R | I | P | M | P |
| | | Y | H | E | N | U | E | E |
| I-23 | 80 | 100 | 100 | 100 | 100 | 100 | 100 | 90 |
| I-23 | 20 | 100 | 100 | 90 | 90 | 90 | 100 | 90 |

| Verbindung | Applikation | A | E | L | S | M | P | S | V |
|---|---|---|---|---|---|---|---|---|---|
| | | L | C | O | E | A | O | T | E |
| | ga.i./ha | O | H | L | T | T | L | E | R |
| | | M | C | M | V | I | C | M | P |
| | | Y | G | U | I | N | O | E | E |
| I-39 | 80 | 90 | 90 | 90 | 90 | 90 | 90 | 90 | 90 |
| I-39 | 20 | 90 | 90 | 90 | 90 | 90 | 90 | 90 | 90 |

| Verbindung | Applikation | A | L | A | M | S |
|---|---|---|---|---|---|---|
| | | L | O | B | A | T |
| | ga.i./ha | O | L | U | T | E |
| | | M | M | T | I | M |
| | | Y | U | H | N | E |
| I-47 | 80 | 90 | 90 | 90 | 90 | 90 |
| I-47 | 20 | 90 | 90 | 90 | 90 | 90 |

| Verbindung | Applikation | A | A | A | M | P | V |
|---|---|---|---|---|---|---|---|
| | | L | B | M | A | H | E |
| | ga.i./ha | O | U | A | T | B | R |
| | | M | T | R | I | P | P |
| | | Y | H | E | N | U | E |
| I-55 | 80 | 100 | 100 | 100 | 100 | 100 | 100 |
| I-55 | 20 | 90 | 90 | 100 | 100 | 100 | 90 |

| Verbindung | Applikation | A | L | A | M | P | S | V | V |
|---|---|---|---|---|---|---|---|---|---|
| | | L | O | M | A | H | T | E | I |
| | ga.i./ha | O | L | A | T | B | E | R | O |
| | | M | M | R | I | P | M | P | T |
| | | Y | U | E | N | U | E | E | R |
| I-61 | 80 | 100 | 100 | 100 | 100 | 100 | 100 | 90 | 90 |
| I-61 | 20 | 100 | 90 | 90 | 100 | 90 | 90 | 90 | 90 |

| Verbindung | Applikation | A | S | M | S | V | V |
|---|---|---|---|---|---|---|---|
| | | L | E | A | T | E | I |
| | ga.i./ha | O | T | T | E | R | O |
| | | M | V | I | M | P | T |
| | | Y | I | N | E | E | R |
| I-67 | 80 | 100 | 90 | 100 | 100 | 100 | 90 |
| I-67 | 20 | 100 | 90 | 90 | 90 | 100 | 90 |

| Verbindung | Applikation | A | E | A | M | P | S | V | V |
|---|---|---|---|---|---|---|---|---|---|
| | | L | C | M | A | O | T | E | I |
| | ga.i./ha | O | H | A | T | L | E | R | O |
| | | M | C | R | I | C | M | P | T |
| | | Y | G | E | N | O | E | E | R |
| I-75 | 80 | 90 | 100 | 100 | 100 | 100 | 100 | 100 | 90 |
| I-75 | 20 | 90 | 90 | 90 | 100 | 90 | 90 | 100 | 90 |

| Verbindung | Applikation | A | E | L | A | A | M | P | V | V |
|---|---|---|---|---|---|---|---|---|---|---|
| | | L | C | O | B | M | A | O | E | I |
| | ga.i./ha | O | H | L | U | A | T | L | R | O |
| | | M | C | M | T | R | I | C | P | T |
| | | Y | G | U | H | E | N | O | E | R |
| I-76 | 80 | 100 | 100 | 90 | 90 | 100 | 100 | 90 | 100 | 100 |
| I-76 | 20 | 90 | 90 | 90 | 90 | 90 | 100 | 90 | 100 | 90 |

| Verbindung | Applikation | A | L | A | A | M | P | V | V |
|---|---|---|---|---|---|---|---|---|---|
| | | L | O | B | M | A | O | E | I |
| | ga.i./ha | O | L | U | A | T | L | R | O |
| | | M | M | T | R | I | C | P | T |
| | | Y | U | H | E | N | O | E | R |
| I-83 | 80 | 90 | 90 | 90 | 90 | 90 | 90 | 90 | 90 |
| I-83 | 20 | 90 | 90 | 90 | 90 | 90 | 90 | 90 | 90 |

| Verbindung | Applikation | A | E | L | M | S | V | V |
|---|---|---|---|---|---|---|---|---|
| | | L | C | O | A | T | E | I |
| | ga.i./ha | O | H | L | T | E | R | O |
| | | M | C | M | I | M | P | T |
| | | Y | G | U | N | E | E | R |
| I-101 | 80 | 90 | 90 | 90 | 90 | 100 | 90 | 90 |
| I-101 | 20 | 90 | 90 | 90 | 90 | 90 | 90 | 90 |

| Verbindung | Applikation | A | E | L | A | M | S | V | V |
|---|---|---|---|---|---|---|---|---|---|
| | | L | C | O | M | A | T | E | I |
| | ga.i./ha | O | H | L | A | T | E | R | O |
| | | M | C | M | R | I | M | P | T |
| | | Y | G | U | E | N | E | E | R |
| I-107 | 80 | 90 | 90 | 90 | 90 | 90 | 100 | 100 | 90 |
| I-107 | 20 | 90 | 90 | 90 | 90 | 90 | 100 | 90 | 90 |

| Verbindung | Applikation | A | E | L | S | M | P | S | V |
|---|---|---|---|---|---|---|---|---|---|
| | | L | C | O | E | A | O | T | E |
| | ga.i./ha | O | H | L | T | T | L | E | R |
| | | M | C | M | V | I | C | M | P |
| | | Y | G | U | I | N | O | E | E |
| Ia-9 | 80 | 90 | 90 | 100 | 90 | 90 | 90 | 90 | 90 |
| Ia-9 | 20 | 90 | 90 | 100 | 90 | 90 | 90 | 90 | 90 |

| Verbindung | Applikation | A | E | L | A | M | P | S | V |
|---|---|---|---|---|---|---|---|---|---|
| | | L | C | O | B | A | O | T | E |
| | ga.i./ha | O | H | L | U | T | L | E | R |
| | | M | C | M | T | I | C | M | P |
| | | Y | G | U | H | N | O | E | E |
| Ia-10 | 80 | 100 | 90 | 100 | 90 | 90 | 90 | 90 | 90 |
| Ia-10 | 20 | 100 | 90 | 90 | 90 | 90 | 90 | 90 | 90 |

In diesem Test zeigen beispielsweise die Verbindungen gemäß Herstellungsbeispiel I-1, I-9, I-17, I-23, I-39, I-47, I-55, I-61, I-67 , I-75, I-76, I-83, I-101 und I-107 sowie Ia-9 und Ia-10 sehr starke Wirkung gegenüber verschiedenen mono- und dikotyledonen Unkräutern.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf.

Nachfolgend sind beispielhaft die Wirksamkeiten einiger beanspruchter Verbindungen auf verschiedene getestete Unkräuter in % dargestellt.

Es bedeuten:

| | | |
|---|---|---|
| 0% | = | keine Wirkung (wie unbehandelte Konrolle) |
| 100% | = | totale Vernichtung |

| Verbindung | Applikation | A | L | M |
|---|---|---|---|---|
| | | L | O | A |
| | g a.i./ha | O | L | T |
| | | M | M | I |
| | | Y | U | N |
| I-1 | 80 | 90 | 90 | 90 |
| I-1 | 20 | 90 | 90 | 90 |

| Verbindung | Applikation | A | L | V |
|---|---|---|---|---|
| | | L | O | I |
| | ga.i./ha | O | L | O |
| | | M | M | T |
| | | Y | U | R |
| I-9 | 80 | 90 | 90 | 90 |
| I-9 | 20 | 90 | 90 | 90 |

| Verbindung | Applikation | A | M | V | V |
|---|---|---|---|---|---|
| | | L | A | E | I |
| | g a.i./ha | O | T | R | O |
| | | M | I | P | T |
| | | Y | N | E | R |
| I-17 | 80 | 100 | 100 | 100 | 100 |
| I-17 | 20 | 90 | 90 | 100 | 100 |

| Verbindung | Applikation | A | A | M | V | V |
|---|---|---|---|---|---|---|
| | | L | M | A | E | I |
| | g a.i./ha | O | A | T | R | O |
| | | M | R | I | P | T |
| | | Y | E | N | E | R |
| I-23 | 80 | 100 | 90 | 100 | 100 | 100 |
| I-23 | 20 | 100 | 90 | 90 | 90 | 100 |

| Verbindung | Applikation | A | M | V |
|---|---|---|---|---|
| | | L | A | E |
| | g a.i./ha | O | T | R |
| | | M | I | P |
| | | Y | N | E |
| I-29 | 80 | 90 | 90 | 90 |
| I-29 | 20 | 90 | 90 | 90 |

| Verbindung | Applikation | A | L | V |
|---|---|---|---|---|
| | | L | O | E |
| | g a.i./ha | O | L | R |
| | | M | M | P |
| | | Y | U | E |
| I-39 | 80 | 90 | 90 | 90 |
| I-39 | 20 | 90 | 90 | 90 |

| Verbindung | Applikation | L | M | V |
|---|---|---|---|---|
| | | O | A | E |
| | g a.i./ha | L | T | R |
| | | M | I | P |
| | | U | N | E |
| I-47 | 80 | 90 | 90 | 100 |
| I-47 | 20 | 90 | 90 | 90 |

| Verbindung | Applikation | A | M | V | V |
|---|---|---|---|---|---|
| | | M | A | E | I |
| | g a.i./ha | A | T | R | O |
| | | R | I | P | T |
| | | E | N | E | R |
| I-55 | 80 | 90 | 100 | 100 | 100 |
| I-55 | 20 | 90 | 90 | 100 | 90 |

| Verbindung | Applikation | S | V | V |
|---|---|---|---|---|
| | | T | E | I |
| | g a.i./ha | E | R | O |
| | | M | P | T |
| | | E | E | R |
| I-67 | 80 | 90 | 100 | 90 |
| I-67 | 20 | 90 | 90 | 90 |

| Verbindung | Applikation | A | M | V |
|---|---|---|---|---|
| | | L | A | E |
| | g a.i./ha | O | T | R |
| | | M | I | P |
| | | Y | N | E |
| I-83 | 80 | 90 | 90 | 100 |
| I-83 | 20 | 90 | 90 | 90 |

| Verbindung | Applikation | A | V |
|---|---|---|---|
| | | L | E |
| | ga.i./ha | O | R |
| | | M | P |
| | | Y | E |
| I-101 | 80 | 90 | 90 |
| I-101 | 20 | 90 | 90 |

| Verbindung | Applikation | V | V |
|---|---|---|---|
| | | E | I |
| | ga.i./ha | R | O |
| | | P | T |
| | | E | R |
| I-107 | 80 | 90 | 90 |
| I-107 | 20 | 90 | 90 |

| Verbindung | Applikation | A | E | L | A | M | V | V |
|---|---|---|---|---|---|---|---|---|
| | | L | C | O | M | A | E | I |
| | ga.i./ha | O | H | L | A | T | R | O |
| | | M | C | M | R | I | P | T |
| | | Y | G | U | E | N | E | R |
| Ia-9 | 80 | 90 | 100 | 100 | 90 | 90 | 90 | 90 |
| Ia-9 | 20 | 90 | 100 | 90 | 90 | 90 | 90 | 90 |

| Verbindung | Applikation | A | E | L | S | M | V | V |
|---|---|---|---|---|---|---|---|---|
| | | L | C | O | E | A | E | I |
| | ga.i./ha | O | H | L | T | T | R | O |
| | | M | C | M | V | I | P | T |
| | | Y | G | U | I | N | E | R |
| Ia-10 | 80 | 90 | 100 | 100 | 90 | 90 | 90 | 90 |
| Ia-10 | 20 | 90 | 90 | 90 | 90 | 90 | 90 | 90 |

Es bedeuten:

| | | |
|---|---|---|
| ALOMY | = | Alopecurus agrestis |
| ECHCG | = | Echinochloa crus-galli |
| LOLMU | = | Lolium multiflorum |
| SETVI | = | Seteria viridis |
| AMARE | = | Amaranthus retroflexus |
| MATIN | = | Matricaria indora |
| STEME | = | Stellaria media |
| VERPE | = | Veronica persica |
| VIOTR | = | Viola tricolor |

In diesem Test zeigen beispielsweise die Verbindungen gemäß Herstellungsbeispiel I-1, I-9, I-17, I-23, I-29, I-39, I-47, I-55, I-67, I-83, I-101 und I-107 sowie Ia-9 und Ia-10 sehr starke Wirkung gegenüber verschiedenen mono- und dikotyledonen Unkräutern.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Verbindungen in unterschiedlichen Dosierungen besprüht. Vier bis fünf Wochen nach der Applikation und Standzeit im Gewächshaus wurde mittels optischer Bonitur festgestellt, dass die erfindungsgemäßen Verbindungen teilweise eine hohe Selektivität zeigen und sich deshalb zur Bekämpfung von unerwünschtem Pflanzenwuchs in landwirtschaftlichen Kulturen eignen.

## Patentansprüche

1. 4-Cyclopropyl-substituierte Phenylsulfonylamino(thio)carbonyltriazolinone der allgemeinen Formel (I), 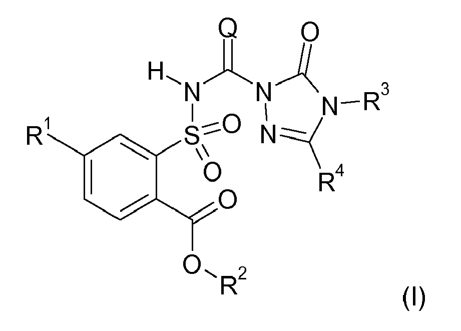 in welcher
R¹ für (R^{c})ₙ-Cyclopropyl steht, wobei der Cyclopropyl-Rest über die Position 1' mit dem Phenyl-Rest verknüpft ist, (R^{c})ₙ an den Positionen 1', 2' und 3' steht und wobei es sich, für alle n ≥ 2, bei R^{c} um gleiche oder verschiedene Reste handeln kann;
R^{c} ausgewählt ist aus der Gruppe, bestehend aus Halogen, Cyano und jeweils gegebenenfalls substituiertem Alkyl, Cycloalkyl, Alkenyl, Alkinyl, Alkoxy, Alkylcarbonyl oder Alkoxycarbonyl;
R² ausgewählt ist aus der Gruppe, bestehend aus jeweils gegebenenfalls substituiertem Alkyl, Alkenyl, Alkinyl, Cycloalkyl, Cycloalkylalkyl, Aryl, Arylalkyl, Heterocyclyl oder Heterocyclylalkyl;
R³ ausgewählt ist aus der Gruppe, bestehend aus Wasserstoff, Hydroxy, Amino, Cyano, (C₂-C₁₀)-Alkylidenamino, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertem Alkyl mit 1 bis 6 Kohlenstoff- atomen, jeweils gegebenenfalls durch Fluor, Chlor und/oder Brom substituiertem Alkenyl oder Alkinyl mit jeweils 2 bis 6 Kohlenstoffatomen, jeweils gegebenenfalls durch Fluor, Chlor, Brom, Cyano, (C₁-C₄)-Alkoxy oder (C₁-C₄)-Alkoxycarbonyl substituiertem Alkoxy, Alkylamino oder Alkyl-carbonylamino mit jeweils 1 bis 6 Kohlenstoffatomen in der Alkylgruppe, Alkenyloxy mit 3 bis 6 Kohlenstoffatomen, Dialkylamino mit jeweils 1 bis 4 Kohlenstoffatomen in den Alkylgruppen, jeweils gegebenenfalls durch Fluor, Chlor, Brom, Cyano und/oder (C₁-C₄)-Alkyl substituiertem Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder jeweils gegebenenfalls durch Fluor, Chlor, Brom, Cyano, Nitro, (C₁-C₄)-Alkyl, Trifluormethyl und/oder (C₁-C₄)-Alkoxy substituiertem Aryl oder Arylalkyl mit jeweils 6 oder 10 Kohlenstoffatomen in der Arylgruppe und gegebenenfalls 1 bis 4 Kohlenstoffatomen im Alkylteil;
R⁴ ausgewählt ist aus der Gruppe, bestehend aus Wasserstoff, Hydroxy, Mercapto, Amino, Cyano, Fluor, Chlor, Brom, Iod, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Alkyl mit 1 bis 6 Kohlenstoffatomen, jeweils gegebenenfalls durch Fluor, Chlor und/oder Brom substituiertes Alkenyl oder Alkinyl mit jeweils 2 bis 6 Kohlenstoffatomen, jeweils gegebenenfalls durch Fluor, Chlor, Cyano, (C₁-C₄)-Alkoxy oder (C₁-C₄)-Alkoxycarbonyl substituiertes Alkoxy, Alkylthio, Alkylamino oder Alkyl-carbonylamino mit jeweils 1 bis 6 Kohlenstoffatomen in der Alkylgruppe, Alkenyloxy, Alkinyloxy, Alkenylthio, Alkinylthio, Alkenylamino oder Alkinylamino mit jeweils 3 bis 6 Kohlenstoffatomen in der Alkenyl- oder Alkinylgruppe, Dialkylamino mit jeweils 1 bis 4 Kohlenstoffatomen in den Alkylgruppen, jeweils gegebenenfalls durch Fluor, Chlor, Brom, Cyano und/oder (C₁-C₄)-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl- bzw. Cycloalkenylgruppe und gegebenenfalls 1 bis 4 Kohlenstoffatomen im Alkylteil oder jeweils gegebenenfalls durch Fluor, Chlor, Brom, Cyano, Nitro, (C₁-C₄)-Alkyl, Trifluormethyl, (C₁-C₄)-Alkoxy und/oder (C₁-C₄)-Alkoxycarbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil;
Q ausgewählt ist aus der Gruppe, bestehend aus Sauerstoff und Schwefel, und
n 0, 1, 2, 3, 4 oder 5 bedeutet,
sowie Salze von Verbindungen der allgemeinen Formel (I).

2. 4-Cyclopropyl-substituierte Phenylsulfonylamino(thio)carbonyltriazolinone nach Anspruch 1, **dadurch gekennzeichnet, dass** Q einem Sauerstoff entspricht.

3. 4-Cyclopropyl-substituierte Phenylsulfonylamino(thio)carbonyltriazolinone nach Anspruch 1 oder 2, **dadurch gekennzeichnet, dass** der Substituent R^{c} ausgewählt ist aus der Gruppe, bestehend aus Chlor, Brom, Fluor, Cyano, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkoxycarbonyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkylcarbonyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₂-C₆)-Alkenyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₂-C₆)-Alkinyl, gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₃-C₆)-Cycloalkyl und gegebenenfalls durch Fluor, Chlor, Brom, Cyano, (C₁-C₄)-Alkyl, (C₁-C₄)-Alkoxy, (C₁-C₄)-Alkoxycarbonyl, (C₁-C₄)-Alkylcarbonyl oder (C₃-C₆)-Cycloalkyl substituiertes (C₁-C₄)-Alkoxy,

4. 4-Cyclopropyl-substituierte Phenylsulfonylamino(thio)carbonyltriazolinone nach einem der Ansprüche 1 bis 3, **dadurch gekennzeichnet, dass** der Substituent R² ausgewählt ist aus der Gruppe, bestehend aus gegebenenfalls durch Fluor, Chlor, Brom, Cyano, (C₁-C₄)-Alkyl, (C₁-C₄)-Alkoxy, (C₁-C₄)-Alkylcarbonyl, (C₁-C₄)-Alkoxycarbonyl oder (C₁-C₃)-Cycloalkyl substituiertes Alkyl mit 1 bis 4 Kohlenstoffatomen, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Alkenyl oder Alkinyl mit jeweils 2 bis 4 Kohlenstoffatomen, gegebenenfalls durch Fluor, Chlor, Brom, Cyano, (C₁-C₄)-Alkoxy, (C₁-C₄)-Alkyl-carbonyl oder (C₁-C₄)-Alkoxycarbonyl substituiertes Cycloalkyl mit 3 bis 6 Kohlenstoffatomen, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Cycloalkylalkyl mit 4 bis 9 Kohlenstoffatomen, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Phenyl oder Benzyl;

5. 4-Cyclopropyl-substituierte Phenylsulfonylamino(thio)carbonyltriazolinone nach einem der Ansprüche 1 bis 4, **dadurch gekennzeichnet, dass** der Substituent R³ ausgewählt ist aus der Gruppe, bestehend Wasserstoff, Hydroxy, Amino, Cyano, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes (C1-C4)-Alkyl, jeweils gegebenenfalls durch Fluor, Chlor, Brom, Cyano, (C₁-C₄)-Alkoxy oder (C₁-C₄)-Alkoxycarbonyl substituiertes (C₁-C₄)-Alkoxy, jeweils gegebenenfalls durch Fluor, Chlor, Brom, Cyano und/oder (C₁-C₄)-Alkyl substituiertes (C₃-C₆)-Cycloalkyl.

6. 4-Cyclopropyl-substituierte Phenylsulfonylamino(thio)carbonyltriazolinone nach einem der Ansprüche 1 bis 5, **dadurch gekennzeichnet, dass** der Substituent R⁴ ausgewählt ist aus der Gruppe, bestehend aus Wasserstoff, Hydroxy, Amino, Cyano, Fluor, Chlor, Brom, Iod, gegebenenfalls durch Fluor, Chlor, Brom, Cyano, (C₁-C₄)-Alkoxy, (C₁-C₄)-Alkylcarbonyl oder (C₁-C₄)-Alkoxycarbonyl substituiertes Alkyl mit 1 bis 4 Kohlenstoffatomen, jeweils gegebenenfalls durch Fluor, Chlor und/oder Brom substituiertes Alkenyl oder Alkinyl mit jeweils 2 bis 4 Kohlenstoffatomen, jeweils gegebenenfalls durch Fluor, Chlor, Cyano, (C₁-C₄)-Alkoxy oder (C₁-C₄)-Alkoxycarbonyl substituiertes Alkoxy, Alkylthio, Alkylamino oder Alkylcarbonylamino mit jeweils 1 bis 4 Kohlenstoffatomen in der Alkylgruppe, Alkenyloxy, Alkinyloxy, Alkenylthio, Alkinylthio, Alkenylamino oder Alkinylamino mit jeweils 3 bis 4 Kohlenstoffatomen in der Alkenyl- oder Alkinylgruppe, jeweils gegebenenfalls durch Fluor, Chlor, Brom, Cyano und/oder (C₁-C₄)-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 4 Kohlenstoffatomen in der Cycloalkyl- bzw. Cycloalkenylgruppe und gegebenenfalls 1 bis 4 Kohlenstoffatomen im Alkylteil oder jeweils gegebenenfalls durch Fluor, Chlor, Brom, Cyano, Nitro, (C₁-C₄)-Alkyl, Trifluormethyl, (C₁-C₄)-Alkoxy und/oder (C₁-C₄)-Alkoxycarbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil;

7. 4-Cyclopropyl-substituierte Phenylsulfonylamino(thio)carbonyltriazolinone nach einem der Ansprüche 1 bis 6, **dadurch gekennzeichnet, dass** der Index n für 0, 1, 2 oder 3 steht.

8. Verfahren zur Herstellung von 4-Cyclopropyl-substituierte Phenylsulfonylamino(thio)carbonyltriazolinone gemäß einem der Ansprüche 1 bis 7, **dadurch gekennzeichnet, dass** man 4-Cyclopropyl-substituierte Phenylsulfonamide der allgemeinen Formel (II), 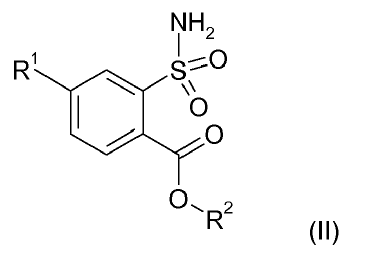 wobei die Substituenten R¹ und R² die in Anspruch 1 genannten Bedeutungen aufweisen,
mit einem heterocyclischen (Thio)Carbonsäurederivat der Formel (III), 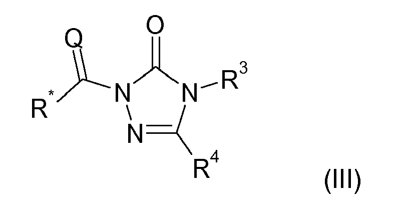 wobei die Substituenten Q, R³ und R⁴ die in Anspruch 1 genannten Bedeutungen aufweisen,
gegebenenfalls in Gegenwart eines Reaktionshilfsmittels, insbesondere Säureakzeptors und gegebenenfalls in Gegenwart eines Verdünnungsmittels umsetzt,
wobei
R* für Halogen oder einen unsubstituierten oder substitierten (C₁-C₂₀)-Kohlenwasserstoffoxyrest wie unsubstituiertes oder substituiertes Alkoxy, Aryloxy, Arylalkoxy oder Alkylaryloxy steht, wobei bevorzugte Substituenten für die vier letztgenannten Reste Halogen, (C₁-C₄)-Alkyl, (C₁-C₄)-Alkyloxy und (C₁-C₄)-Halogenalkyl sind, vorzugsweise steht R* für Fluor, Chlor, Brom, (C₁-C₆)-Alkoxy, (C₆-C₁₀)-Aryloxy, (C₆-C₁₀)-Aryl-(C₁-C₆)-alkoxy oder (C₁-C₆)-Alkyl-(C₆-C₁₀)-aryloxy steht.

9. Verbindungen der allgemeinen Formel (II) 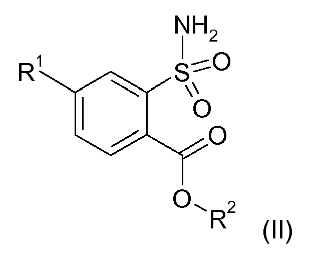 wobei die Substituenten R¹ und R² die Bedeutung gemäß Anspruch 1 haben.

10. Zusammensetzungen, enthaltend mindestens eine Verbindung der 4-Cyclopropyl-substituierten Phenylsulfonylamino(thio)carbonyltriazolinone der allgemeinen Formel (I) gemäß einem der Ansprüche 1 bis 7.

11. Zusammensetzung nach Anspruch 10, **dadurch gekennzeichnet, dass** die Zusammensetzung mindestens einen weiteren Wirkstoff umfasst, welcher ausgewählt ist aus der Gruppe, bestehend aus mindestens einem weiteren Herbizid und mindestens einem Safener.

12. Verwendung der 4-Cyclopropyl-substituierten Phenylsulfonylamino(thio)-carbonyltriazolinone der allgemeinen Formel (I) gemäß einem der Ansprüche 1 bis 7 als Herbizide und Pflanzenwachstumsregulatoren.

13. Verwendung der Zusammensetzungen gemäß einem der Ansprüche 10 oder 11 als Herbizide und Pflanzenwachstumsregulatoren.

14. Verwendung nach Anspruch 12 oder 13 zur Pflanzenbekämpfung in speziellen Pflanzenkulturen oder als Pflanzenschutzregulator.
